# EUROPEAN PATENT SPECIFICATION

(11) **EP 1 223 197 B1**
(45) Date of publication and mention of the grant of the patent: **08.08.2012**
(21) Application number: 01130643.8
(22) Date of filing: 21.12.2001
(51) Int. Cl.: C09B 23/06, C09B 23/08

(54) **Symmetric, monofunctionalised polymethine dyes labelling reagents**
Symmetrische, monofunktionalisierte Polymethinfarbstoffe als Markierungsreagenzien
Colorants polyméthiniques symétriques et monofonctionnalisés comme réactifs de marquage

(30) Priority: 03.01.2001 EP 01100260
(43) Date of publication of application: 17.07.2002
(73) Proprietor: Visen Medical, Inc., Waltham MA 02451 (US)
(72) Inventor: Caputo, Giuseppe, 10125 Torino (IT); Della Ciana, Leopoldo, 10015 Ivrea (Torino) (IT)
(74) Representative: Kirkham, Nicholas Andrew

(56) References cited:
- EP-A- 0 670 374
- EP-A- 0 753 584
- DE-A- 19 911 102
- US-A- 5 268 486
- PATENT ABSTRACTS OF JAPAN vol. 018, no. 462 (C-1243), 29 August 1994 (1994-08-29) & JP 06 145539 A (FUJI PHOTO FILM CO LTD), 24 May 1994 (1994-05-24)

## Description

Polymethine dyes, also known as cyanines, conform to the generalised formula:

X-(CR)ₙ-X¹

in which n is an odd positive integer and (n+3) n electrons are distributed over the polymethine chain and the terminal atoms X and X¹; R, attached to the methine carbon C, is hydrogen or a radical. In the large majority of dyes X and X¹ are the nitrogen atoms in a heterocyclic ring, but dyes are known in which one or both of the groups art non-cyclic or carbocyclic. The -CR- groups can be replaced by one ore more aza (-N=) links.

EP-A-0 753 854 discloses sequencing near infrared and infrared fluorescent labelled DNA for detecting using laser diodes and suitable labels therefor.

EP-A-0 670 374 discloses a method of identifying strands of DNA using infrared fluorescent labels.

JP-A-6 145 539 discloses particular cyanine compounds.

Comprehensive reviews regarding polymethine dyes have been by written by L. G.S. Brooker, "The Theory of the Photographic Process" Mees Ed., Macmillan, New York, (1942), p. 987 and (1966), p. 198; Frances M. Hamer, in "The Chemistry of Heterocyclic Compounds", Vol 18, "The Cyanine Dyes and Related Compounds", Weissberger, Ed, Wiley Interscience, New York, (1964); G.E. Ficken, "The Chemistry of Synthetic Dyes", Vol 4, K. Venkataraman Ed., Academic Press, New York, (1971), p.211; A.I. Kiprianov, Usp. Khim., 29, 1336, (1960), 35, 361 (1966), 40, 594 (1971); D.W. Heseltine, "The Theory of the Photographic Process", 4th edition, James Ed., Macmillan, New York, (1977), chapter 8, "Sensitising and Desensitising Dyes"; S. Daehne, Phot. Sci. Eng., 12, 219 (1979); D.J. Fry, "Rodd's Chemistry of Carbon Compounds", "Cyanine Dyes and Related Compounds", Vol. IVb, chapter 15, p.369 Elsevier, Amsterdam, (1977); Supplement to Vol. IVb, 2nd Edition (1985), p.267; H. Zollinger, "Color Chemistry", VCH, Weinheim (1987), chapters 3 and 14; D. M. Sturmer, "The Chemistry of Heterocyclic Compounds", "Special Topics in Heterocyclic Chemistry", chapter VIII, "Synthesis and Properties of Cyanine and Related Dyes", Weissberger Ed., Wiley, New York, (1977); "The Kirk-Othmer Encyclopaedia of Chemical Technology" Vol 7, p. 782, "Cyanine Dyes", Wiley, New-York, (1993).

For many years, polymethine dyes have been very useful as sensitisers in photography, especially in the red and near infrared regions of the spectrum. However, in more recent years, there has been an upsurge of new uses of these dyes in innovative technological areas, such as laser and electro-optic applications, optical recording media, medical, biological and diagnostic. These new applications of polymethine dyes place high demands on the degree of purity required, and the reproducibility of synthetic methods and purification steps is very important. These requirements are especially stringent for dyes designed to improve detection of ribonucleic acid (RNA), deoxyribonucleic acid (DNA) and of antigens in immunoassays. In these fields, the trend toward an increasing miniaturisation is accompanied by an increasing demand on sensitivity of the reporter molecules or labels. One way to increase the sensitivity of conventional fluorescence method is to use laser sources for the excitation. However, traditional fluorescent labels based on fluoresceins or rhodamins required expensive and/or bulky lasers. Moreover, their fluorescence occurs in the blue-green to green regions of the visible spectrum, where interference from the sample matrix is more likely to occur. Polymethine dyes do not suffer from these limitations. They can be efficiently excited by means of small, inexpensive solid state devices such as laser diodes or light emitting diodes, with extinction coefficients often several times higher than fluoresceins and rhodamines; they emit in the red and near-infrared regions of the spectrum, where non-specific fluorescence from the sample is low or lacking; another sources, Raman noise, becomes smaller with the inverse fourth power of wavelength (Imasaka, T., Yoshitake, A., and Ishibashi, N, "Semiconductor Laser Fluorimetry in the Near-Infrared Region", Anal. Chem., 56, 1077 (1984); Imasaka, T., and Ishibashi, N., "Diode Lasers and practical trace Analysis", Anal. Chem., 62, 363 (1990); Matsuoka, M., Ed., "Infrared Absorbing Dyes", Plenum Press, New York, (1990); J. Fabian, H. Nakazumi, M. Matsuoka, "Near-Infrared Absorbing Dyes", Chem. Rev., 92, 1197, (1992); S. Daehne, U. Resch-Genger, O.S. Wolfbeis, "Near-Infrared Dyes for High Technology Applications", Kluwer Academic Publishers, Dordrecht (1997).

To be useful as a label, a dye has to be provided with a suitable side chain containing a functional group. While the main part of the dye structure is generally known from previous applications, the introduction of a functional group into the structure for the purpose of conjugation, or binding to another molecule, represents the innovative step in the inventions concerning the use of the dye as a labelling reagent. In general, only one such functionalised side arm is preferable, in order to avoid cross-linking or purification problems. With a few exceptions, limited to heptamethine dyes, the standard approach in the design of polymethine labelling reagents has been to attach the functionalised side arm to one of the heterocyclic nuclei of the dye, formula (a):

HET₁-HET₂-Z

See, for instance: J.S. Lindsey, P.A. Brown, and D.A. Siesel, "Visible Light-Harvesting in Covalently-Linked Porphyrin-Cyanine Dyes, Tetrahedron, 45, 4845, (1989); R.B. Mujumdar, L. A. Ernst, S.R. Mujumdar, and A.S. Waggoner, ""Cyanine Dye Labelling Reagents Containing Isothiocyanate Groups", Cytometry, 10, 11 (1989); L.A. Ernst, R.K. Gupta, R.B.Mujumdar, and A.S. Waggoner, "Cyanine Dye Labelling Reagents for sulphydryl Groups", Cytometry, 10, 3, (1989); P.L. Southwick P.L., L.A. Ernst, E. W. Tauriello, S.R. Parker, R.B. Mujumdar, S.R. Mujumdar, H.A. Clever, and A.S. Waggoner, "Cyanine Dye Labelling Reagents-Carboxymethylindocyanine Succinimidyl Esters", Cytometry 11, 418 (1990); R.B. Mujumdar, L.A. Ernst, Swati R. Mujumdar, C.J. Lewis, and A.S. Waggoner, "Cyanine Dye Labelling Reagents: Sulfoindocyanine Succinimidyl Esters", Bioconjugate Chemistry, 4, 105, (1993); A.J.G. Mank, E.J. Molenaar, H.Lingeman, C. Goojer, U. A. Th. Brinkman, and N. H. Velthorst, "Visible Diode Laser Induced Fluorescence Detection in Liquid Chromatography after Precolumn Derivatisation of Thiols", Anal. Chem., 65, 2197, (1993); H. Yu., J. Chao, D. Patek, S.R. Mujumdar, and A.S. Waggoner, "Cyanine dye dUTP analogs for enzymatic labelling of DNA Probes", Nucl. Acids Res 22, 3226, (1994); Z. Zho, J. Chao, H. Yu, and A.S. Waggoner,, "Directly labelled DNA probes using fluorescent nucleotides with different length linkers", Nucl. Acids, Res, 22, 3226. A.J.G. Mank, H.T.C. van der Laan, H.Lingeman, Cees Goojer, U. A. Th. Brinkman, and N. H. Velthorst, "Visible Diode Laser-Induced Fluorescence Detection in Liquid Chromatography after Precolumn Derivatisation of Amines", Anal. Chem., 67, 1742, (1995); S.R. Mujumdar, R.B. Mujumdar, C.M. Grant, and A.S. Waggoner, "Cyanine Labelling Reagents: sulfobenzoindocyanine succinimidyl esters", Bioconjugate Chemistry, 7, 356, (1996). Patent Literature: P.L. Southwick, and A.S. Waggoner, "Intermediate for and Fluorescent Cyanine Dyes containing Carboxylic Acid Groups", U.S. Patent No. 4981977, Jan 1, 1991; A.S. Waggoner, L.A. Ernst, and Mujumdar, R.B., "Method for Labelling and Detecting Materials Employing Arylsulfonate Cyanine Dyes", U.S. Patent No. 5268486, Dec. 7., 1993; A.S. Waggoner, "Cyanine Dyes as Labelling Reagents for Detection of Biological and Other Materials by Luminescence Methods", U.S. Patent No. 5627027, May 6, 1996; A.S. Waggoner, and R.B. Mujumdar, "Rigidised Trimethine Cyanine . Dyes", WO99/311181; G.-Y. Shen, T.S. Dobashi, "Cyanine Dye Activating Group with Improved Coupling Selectivity"; T. S. G. M. Little, R. Raghavachari; N. Narayanan; H.L. Osterman, "Fluorescent Cyanine Dyes", U.S. Patent No. 6,027,709, Feb 22, 2000.

The general synthetic strategy necessary to prepare these labelling reagents is as follows. First, a quaternised nitrogen heterocycle HET₁ is prepared. Then, this heterocyclic base is reacted with an electrophilic reagent such as PhNH-(CH=CH)ₙ-CH=NHPh·HCl or RO-(CH=CH)ₙ-CH(OR)₂, where Ph is a phenyl ring and R a methyl or ethyl group, to obtain a so-called hemicyanine dye, HET₁-(CH=CH)ₙNHPh or HET₁-(CH=CH)ₙNAcPh, where Ac is the acetyl radical, or HET₁-(CH=CH)ₙ-OR. These intermediates are then reacted with a different quaternary nitrogen heterocycle, HET₂. The functionalised side arm can be attached either to the first or to the second quaternised nitrogen heterocycle. The final result is an asymmetric polymethine labelling reagent, HET₁-(CH=CH)ₙ-HET₂-Z.

Unfortunately, the hemicyanine intermediates are notoriously difficult to obtain in good yields and/or in a pure form (see, for example, F. M. Hamer, "Some Unsymmetrical Pentame-thincyanine Dyes and their Tetramethin Intermediates", J. Chem. Soc., 32 (1949) and R.B. Mujumdar, L.A. Ernst, Swati R. Mujumdar, C.J. Lewis, and A.S. Waggoner, "Cyanine Dye Labelling Reagents: Sulfoindocyanine Succinimidyl Esters", Bioconjugate Chemistry, 4, 105, (1993); in particular, note that when Mank (Anal. Chem., 67, 1744) tried to synthesise an asymmetric dicarbocyanine label described in the previous reference he obtained a total yield of 18% of dicarbocyanines, from which the desired product was difficult to separate; therefore he devised an alternative approach based on 1,3,3-trimethoxypropene. Unfortunately, this chemical is no longer available commercially.

In order to avoid such difficulties, the present invention is based on an alternative approach to the design of polymethine dyes with a single functionalised side arm. This general approach is illustrated in formula (b), below:

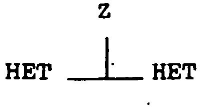

In this case, the functionalised side arm Z is attached to the centre of the dye molecule, resulting in a symmetric labelling reagent. It is immediately obvious from this scheme that only one type of heterocyclic base, HET, is necessary and that the dye can be synthesised in one step, from HET (2 equivalents) and an electrophilic reagent bearing the functionalised side arm. The overall result is a much more convergent, more efficient synthesis of the required labels.

However, thus far, this approach has only found very limited application: the main example is in the synthesis of an isothiocyanate derivative of an heptamethine dye: L. Strekowski, M. Lipowska, and G. Patonay, "Facile Derivatisation of Heptamethine Cyanine Dyes", Synth. Comm., 22(17), 2593-2598 (1992); L. Strekowski, M. Lipowska, and G. Patonay, "Substitution Reactions of a Nucleofugal Group in Heptamethine Cyanine Dyes. Synthesis of an Isothiocyanate Derivative for Labelling of Proteins with a Near-Infrared Chromophore" J. Org. Chem. 57, 4578, (1992); N. Narayan, and G. Patonay, "A New Method for the Synthesis of Heptamethine Cyanine Dyes", J. Org. Chem. 60, 2391-2395, (1995). No example was given in the case of pentamethine labelling reagents and only one example for their trimethine analogues (Compound IX in WO99/311181).

The present inventors succeeded in synthesising symmetric monofunctionalised polymethine dyes as shown in formula (b) having a wide variety of functional groups other than isothiocyanates and also having different functionalised arm chain lengths, which can be used for the labelling of a wide range of analytically and diagnostically useful biomolecules.

Examples of such analytically and diagnostically useful biomolecules include, but are not limited to, nucleotides and nucleosides, oligonucleotides, vitamins, proteins such as for example antibodies, antigens, streptavidin, and the like.

### Detailed description of the invention

The dyes of the present invention are defined in the claims.

The dyes of the present invention are obtained by reacting 2 moles of quaternised nitrogen heterocycle base, HET, with suitable electrophilic reagents, such as diphenylformamidines or trialkylorthoformates and their vinilogs. The functionalised side arm can either be attached to the electrophilic reagent in a previous step, or after the formation of the polymethine dye structure.

The quaternised heterocyclic nuclei, HET, are commercially available, or can be synthesised by known methods from commercially available precursors. For example, the following heterocycle bases are all commercially available: 2,3,3-trimethyl-3-H-indole, 1,1,2-trimethyl-1-H-benz(e)indole, 2-methylbenzothiazole, 2-methylbenzoxazole,2-methylnaphth[1,2-d]thiazole, 2-methylnaphth[1,2-d] oxazole, 2-methyl- naphth [2,1-d]oxazole.

Other heterocyclic nuclei can be synthesised by known methods. For example, sulphonated indoles can be made from the corresponding aminosulphonic acids: these compounds are first converted to the corresponding hydrazinosulphonic acids by diazotisation followed by reduction with tin (II) chloride or other reducing agents, especially SO₂ and sulphites; in the next step the hydrazine intermediates were condensed with 2-methylbutanone to yield the corresponding indoles, and then alkylated at the nitrogen with alkyl halides or sultones. Sulphonated benzo- and naphthoxazoles were obtained from the corresponding aminophenol and aminonaphtolsulphonic acids by condensation with acetic anhydride. A similar approach was used to prepare sulphonated benzo- and naphthothiazoles. 2-methyl-naphth[2,1-d]thiazole, was similarly prepared by condensation with acetic anhydride.

N,a-alkylene cyclammonium salts of 3,3-dimethyl-3-H-indole, 1,1,2-dimethyl-1-H-benz(e)indole and their sulphonated analogues, were obtained by following the methods disclosed by L.L. Lincoln and D.W. Heseltine in "Merocyanine Sensitisers for Silver Halide", U.S. Patent No. 3,282,932 (1966), L.L.Lincoln and L.G.S. Brooker in "Photographic Sensitising Dyes of the Merocyanine And Styryl Types in Silver Halide Photographic Emulsions", U.S. Patent No. 3,397,981 (1968); G.L. Oliver in "Photographic Silver Halide Emulsions Containing N,α-alkylene Bridged Merocyanine Sensitising Dyes", U.S. Patent 3,403,026 (1968); G.L. Oliver, in "Silver Halide Emulsions Containing N,α-alkylene Bridged Indocyanine Sensitising Dyes", U.S. Patent No. 3,408,195 (1968). N,α-alkylene cyclammonium salts of benzothiazole, benzoxazole, naphth[1,2-d]thiazole, naphth[2,1-d]oxazole, naphth[1,2-d]oxazole, naphth[2,1-d]oxazole, and their sulphonated analogs were prepared according to the methods described by F.DS. Babichev, and N. Ya Derkach, Ukr. Khim. Zh.,22, 208 (1956); F.S. Babichev, and Neplyuev, "Benzothiazolylalkyl Carboxylic Acids and their Derivatives-IV-Benzothiazolylalkyl Carbinols" , Zh. Obsch. Khim., 32, 857 (1962); ); F.S. Babichev, and Neplyuev, "Benzothiazolylalkyl Carboxylic Acids and their Derivatives -V-2,3-Polymethylenebenzothiazolium Salts" , Zh. Obsch. Khim., 32, 860(1962); F.S. Babichev "Condensation of o-Aminobenzenethiol with Lactones", Zh. Obsch. Khim., 33, 3016, (1963).

Methods for the synthesis of 1-Alkyl-2-methyl-benzo[c,d]indole nuclei, were provided by Ya. B. Shteinberg, in the article "Benzo[c,d]indocyanines", Khim. Geterotsik. Soedin. 3, 340 (1973) and by F.A. Mikhailenko, N.P. Vasilenko, A.D. Kachkovskii and Yu. I. Rozhinskii in "Effect of Polar Substituents and the Length of the Polymethine Chain on the Color of Cyanine Dyes of the Benzo[c,d]indole Series", Zh. Org. Khim., 18, 435 (1982).

In the assembly of the dyes of this invention, two identical molecules of the above described quaternised nitrogen heterocycles are condensed with one molecule of electrophilic intermediates, which provide the bridging methine carbon atoms. For example, N,N-diphenylformamidines or trialkylorthoformates each add one methine carbon atom to the polymethine chain, giving rise to trimethincyanines or carbocyanines; malonaldehyde dianils or trialkoxypropenes contribute three methines to pentamethincyanines, or dicarbocyanines; and glutaconaldehyde dianils introduce five methines, to produce heptamethincyanines or tricarbocyanines, and so on. In one aspect of this invention, the functionalised side arm needed for linking the dye to another molecule, can be inserted into the middle, or meso (µ), position of the polymethine chain either before, or after the synthesis of the cyanine skeleton. Especially useful for this purpose are certain electrophilic reagents bearing a halogen atoms at the meso position, or cyanine dyes with halogens attached at this position. For example, meso-chloro- or bromomalonaldehyde dianils can be made from mucochloric or mucobromic acids by treatment with ethanol and aniline hydrochloride according to the directions given in Dieckman and Platz, Berichte 4639 (1904). These compounds can be used to synthesise the corresponding meso-chloro, or bromodicarbocyanines. Similarly, the Vilsmeier-Haack-Arnold reaction can be used to prepare cyclic analogs of glutaconaldehyde bearing a halogen atom in the meso position: this structure is then incorporated into the corresponding cyanine dye. For example, S. M. Makin, L.I. Boiko and O.A. Shavrygina describe the synthesis of (5-phenylamino-2,4-trimethylene-3-chloro-2,4-pentadienylidene)phenylammonium chloride from cyclohexanone and the complex formed by mixing N,N-dimethylformamide and phosphorus oxychloride, "Aminoformylation of Unsaturated Aldehydes, 2-Alkoxyaldehydes and their Acetals, and Ketones of the Alicyclic Series", Zh. Org. Khim., 13, 1189 (1977). In our hands, the corresponding reaction with cyclopentanone only produced monoaminoformylated derivatives. The corresponding meso-bromo derivatives can be obtained by using phosphorus bromide in place of the oxychloride as shown by A.I. Ponogaev and S.M. Makin in "Meso-bromo-substituted Tricarbocyanines with Cyclic Fragments in the Conjugation Chain", Zh. Org. Khim. 17, 167 (1980). These methods were applied successfully to 2-indanone by G.A. Reynolds and K.H. Drexhage, in "Stable Heptamethine Pyrylium Dyes that Absorb in the Infrared", J. Org. Chem, 42, 885 (1977) and by G.M. Sosnovskii, A.P. Lugovskii, and I.G. Tishchenko in "Synthesis of Meso-substituted Tricarbocyanine Dyes with an Ortho-phenylene Bridge in the Chromophore", Zh. Org. Khim. 19, 2143 (1983). Also, in the later article, methods are described for the introduction of a phenyl substituent in the meso-position or the substituted cyclic glutaconaldehyde intermediates: the cycloalkanones are reacted with PhMgBr or PhLi yielding the corresponding alcohols which can be easily dehydrated; these intermediates are subjected to a two-step aminoformylation, first with dimethylformamide dimethyl acetal and then with the DMF-POCl₃ complex. Functionalised side arms can be introduced in the *para* position of the phenyl substituent, by masking the functionality with appropriate protective groups, i.e. dioxanes or dioxolanes for aldehydes, oxazolines for carboxylic groups and tetrahydropyranes for alcohols, as described in the book by T. W. Greene and P.G.M. Wuts, "Protective Groups in Organic Synthesis", John Wiley & Sons, New York, NY (1991).

Trimethincyanines with a meso chloro groups are best made as disclosed by G. L. Oliver in "Photographic Emulsions and µ-chlorocarbocyanine Dyes", U.S. Patent 3,656,960 (1962).

In trimethincyanines and heptamethine cyanines the meso halogen is easily displaceable by more nucleophilic atoms such as O, S, Se, and N. This provides a convenient route for the introduction of a great number of functionalised side arms, by using reagents bearing a functional group at their distal end. As was shown above, this method found some very limited application by Patonay and his group, in the preparation of heptamethincyanines with an isothiocyanate reactive group at the distal end of a thiophenyl meso substituent. This reagent has only very limited utility, mostly for cell or protein labelling, while it is totally unsuitable for the labelling of small molecules such as nucleotides. It is the purpose of this invention to provide better compounds by this route for applications where it is important to limit the perturbation caused to much the labelled molecule.

The above method could not be extended to pentamethincyanines, where the meso halogen not easily displaceable by nucleophiles. This effect is due to the alternation of charge density in the meso methine carbon in the series tri-, penta- and heptamethine dyes. In other words, the halogen-carbon bond in the meso position of pentamethincyanines is similar to that found in vinyl or aromatic halides. On this basis, it is possible to exploit the methods developed for the creation of carbon-carbon bonds from sp² halides and unsaturated hydrocarbons with the help of palladium catalysts, as described by R.F. Heck in "Palladium Reagents in Organic Synthesis", Academic Press, New York, NY, 1985; J. Tsuji, in "Palladium Reagents and Catalysts", John Wiley & Sons, New York, NY, 1995; and by J.-L. Malleron, J.-C- Fiaud, and J.-Y. Legros, in "Handbook of Palladium-Catalysed Organic Reactions", Academic Press, New York, NY, 1997. Especially useful in our context is the reaction between sp² halides and alkynes bearing a functional groups. These reactions are tolerant of many functionalities, occur under mild condition and in high yields. Similar methods proved successful also in the case of trimethine and heptamethincyanines, where the meso halogen is much more labile.

Other methods were developed for pentamethincyanines. In some cases, it is possible to synthesise pentamethine cyanines bearing useful functional groups in the meso position, for example an ester group, as disclosed by F.P. Doyle, in "Improvements in or Relating to the Production of Cyanine Dyestuffs and to the Sensitising of Photographic Emulsions", G.B. Patent No. 640,127 (1950). The Vilsmeyer-Haack-Arnold reaction is also very useful for the preparation of meso-substituted malondialdehydes needed for the preparation of the corresponding meso-substituted pentamethincyanines, C.M. Marson and P.R. Giles, "Synthesis Using Vilsmeier Reagents", CRC Press, Boca Raton, FL, 1994. For example by treating substituted acetic acids, R-COOH, where R is aryl, chloro ethoxycarbonyl, with an excess of the Vilsmeier reagent N,N,dimethylformamide-POCl₃ results in substituted malonaldialdehyde synthetic equivalents, CH₃N⁺=C-CR=CH-N(CH₃)₂.

The subject-matter of the present invention is therefore constituted by symmetric cyanine labelling dyes having the general formula (1): 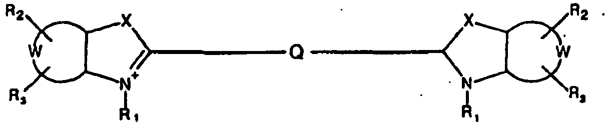 wherein:
X is selected from the group consisting of O, S and C(CH₃)₂;
W represents non-metal atoms required to form a benzo-condensed or a naphtho-condensed ring;
R₁ is selected from the group consisting of (CH₂)ₙCH₃, (CH₂)ₙSO₃⁻ and (CH₂)ₙSO₃H, wherein n is an integer selected from 0 to 6 when R₁ is (CH₂)ₙCH₃, and n is an integer selected from 3 to 6 when R₁ is (CH₂)ₙSO₃- or (CH₂)ₙSO₃H;
R₂ and R₃ are independently selected from the group consisting of H, a sulphonic moiety and a sulphonate moiety; Q is selected from the group consisting of: 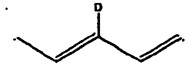 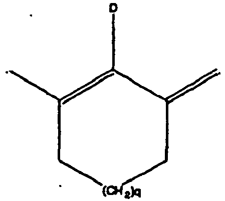 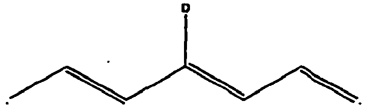 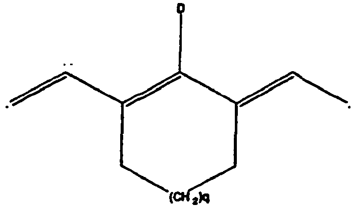 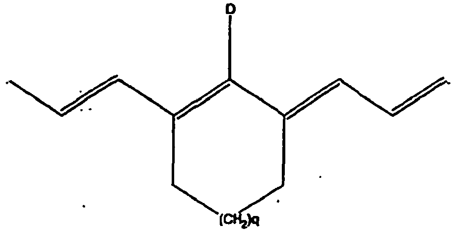
wherein q is 0 or 1 and D is selected from the group consisting of:

-C≡C-G;

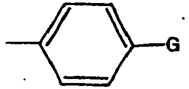

and 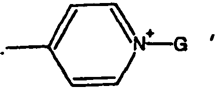 and
wherein
G is a nucleophile moiety selected from the group consisting of (CH₂)ₘNH₂, (CH₂)ₘSH, (CH₂)ₘY(CH₂)ₚOH, (CH₂)ₘY(CH₂)ₚNH₂ and (CH₂)ₘY(CH₂)ₚSH, wherein Y is selected from the group consisting of -NH-, -CONH-, -O- and - S-, m is an integer selected from 0 to 6 and p is an integer selected from 1 to 6;
or wherein G is a moiety capable of reacting with N, O or S nucleophiles, and is selected from the group consisting of (CH₂)ₘCOOH, (CH₂)ₘglycidyl, (CH₂)ₘmaleimide, (CH₂)ₘCO-NHS, (CH₂)ₘCO-imidazole, (CH₂)ₘSO₂CH=CH₂, (CH₂)ₘCONHNH₂, (CH₂)ₘCHO, (CH₂)ₘY(CH₂)ₚCOOH, (CH₂)ₘY(CH₂)ₚglycidyl, (CH₂)ₘY(CH₂)ₚmaleimide, (CH₂)ₘY(CH₂)ₚCO-NHS, (CH₂)ₘY(CH₂)ₚCO-imidazole, CH₂(CH₂)ₘO-PAM, (CH₂)ₘY(CH₂)ₚSO₂CH=CH₂, (CH₂)ₘY(CH₂)ₚCONHNH₂, (CH₂)ₘY(CH₂)ₚCHO, and (CH₂)ₘY(CH₂)ₚO-PAM,
wherein Y, m and p have the meanings indicated above,
wherein

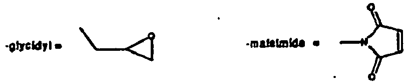

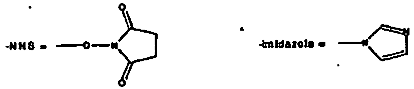

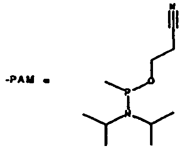

In the above formulae Y is selected from the group consisting of -NH-, -CONH-, -O- and -S-, m is an integer selected from 0 to 6 and p is an integer selected from 1 to 6.

In the above illustrated symmetric cyanines, it is preferred that at least one of the moieties R₁ to R₃ is, or contains a sulfonic or a sulphonate moiety.

In a preferred embodiment of the symmetric cyanines of the invention, X is C(CH₃)₂ and one of the moieties R₂ and R₃ is a sulphonic moiety or a sulphonate moiety; according to this embodiment of the invention, R₁ is more preferably (CH₂)ₙSO₃⁻or (CH₂)ₙSO₃H.

In another preferred embodiment of the symmetric cyanines of the invention, X is S and R₁ is (CH₂)ₙSO₃⁻ or (CH₂)ₙSO₃H.

Also salts of the above illustrated symmetric cyanines are within the scope of the present invention. Examples of such salts include, but are not limited to, chloride, iodide and bromide salts; sodium, potassium and magnesium salts.

Also the valence tautomers of the symmetric cyanines of formula (1) are included within the scope of the invention, wherein the valence tautomerism is intended to mean the shifting of the conjugated bonds in the polymethine chain. Examples of symmetric cyanines of the present invention are compounds of formula (1) in which G, R₁, R₂, R₃ have the meanings illustrated in table 1 below.

**Table 1**

| G | R₁ | R₂ | R₃ |
|---|---|---|---|
| (CH₂)ₘCOOH | (CH₂)ₙCH₃ | H | H |
| (CH₂)ₘCOOH | (CH₂)ₙSO₃H | H | SO₃H |
| (CH₂)ₘCOOH | (CH₂)ₙCH₃ | SO₃H | H |
| (CH₂)ₘCOOH | (CH₂)ₙSO₃H | SO₃H | H |
| (CH₂)ₘCOOH | (CH₂)ₙCH₃ | SO₃H | SO₃H |
| (CH₂)ₘCOOH | (CH₂)ₙSO₃H | SO₃H | SO₃H |
| (CH₂)ₘOH | (CH₂)ₙCH₃ | H | H |
| (CH₂)ₘOH | (CH₂)ₙSO₃H | H | SO₃H |
| (CH₂)ₘOH | (CH₂)ₙCH₃ | SO₃H | H |
| (CH₂)ₘOH | (CH₂)ₙSO₃H | SO₃H | H |
| (CH₂)ₘOH | (CH₂)ₙCH₃ | SO₃H | SO₃H |
| (CH₂)ₘOH | (CH₂)ₙSO₃H | SO₃H | SO₃H |
| (CH₂)ₘNH₂ | (CH₂)ₙCH₃ | H | H |
| (CH₂)ₘNH₂ | (CH₂)ₙSO₃H | H | SO₃H |
| (CH₂)ₘNH₂ | (CH₂)ₙCH₃ | SO₃H | H |
| (CH₂)ₘNH₂ | (CH₂)ₙSO₃H | SO₃H | H |
| (CH₂)ₘNH₂ | (CH₂)ₙCH₃ | SO₃H | SO₃H |
| (CH₃)ₘNH₂ | (CH₂)ₙSO₃H | SO₃H | SO₃H |
| (CH₂)ₘSH | (CH₂)ₙCH₃ | H | H |
| (CH₂)ₘSH | (CH₂)ₙSO₃H | H | SO₃H |
| (CH₂)ₘSH | (CH₂)ₙCH₃ | SO₃H | H |
| (CH₂)ₘSH | (CH₂)ₙSO₃H | SO₃H | H |
| (CH₂)ₘSH | (CH₂)ₙCH₃ | SO₃H | SO₃H |
| (CH₂)ₘSH | (CH₂)ₙSO₃H | SO₃H | SO₃H |
| (CH₂)ₘglycidyl | (CH₂)ₙCH₃ | H | H |
| (CH₂)ₘglycidyl | (CH₂)ₙSO₃H | H | SO₃H |
| (CH₂)ₘglycidyl | (CH₂)ₙCH₃ | SO₃H | H |
| (CH₂)ₘglycidyl | (CH₂)ₙSO₃H | SO₃H | H |
| (CH₂)ₘglycidyl | (CH₂)ₙCH₃ | SO₃H | SO₃H |
| (CH₂)ₘglycidyl | (CH₂)ₙSO₃H | SO₃H | SO₃H |
| (CH₂)ₘmaleimide | (CH₂)ₙCH₃ | H | H |
| (CH₂)ₘmaleimide | (CH₂)ₙSO₃H | H | SO₃H |
| (CH₂)ₘmaleimide | (CH₂)ₙCH₃ | SO₃H | H |
| (CH₂)ₘmaleimide | (CH₂)ₙSO₃H | SO₃H | H |
| (CH₂)ₘmaleimide | (CH₂)ₙCH₃ | SO₃H | SO₃H |
| (CH₂)ₘmaleimide | (CH₂)ₙSO₃H | SO₃H | SO₃H |
| (CH₂)ₘCO-NHS | (CH₂)ₙCH₃ | H | H |
| (CH₂)ₘCO-NHS | (CH₂)nSO₃H | H | SO₃H |
| (CH₂)ₘCO-NHS | (CH₂)ₙCH₃ | SO₃H | H |
| (CH₂)ₘCO-NHS | (CH₂)ₙSO₃H | SO₃H | H |
| (CH₂)ₘCO-NHS | (CH₂)ₙCH₃ | SO₃H | SO₃H |
| (CH₂)ₘCO-NHS | (CH₂)ₙSO₃H | SO₃H | SO₃H |
| (CH₂)ₘCO-imidazole | (CH₂)ₙCH₃ | H | H |
| (CH₂)ₘCO-imidazole | (CH₂)ₙSO₃H | H | SO₃H |
| (CH₂)ₘCO-imidazole | (CH₂)ₙCH₃ | SO₃H | H |
| (CH₂)ₘCO-imidazole | (CH₂)ₙSO₃H | SO₃H | H |
| (CH₂)ₘCO-imidazole | (CH₂)ₙCH₃ | SO₃H | SO₃H |
| (CH₂)ₘCO-imidazole | (CH₂)ₙSO₃H | SO₃H | SO₃H |
| (CH₂)ₘSO₂CH=CH₂ | (CH₂)ₙCH₃ | H | H |
| (CH₂)ₘSO₂CH=CH₂ | (CH₂)ₙSO₃H | H | SO₃H |
| (CH₂)ₘSO₂CH=CH₂ | (CH₂)ₙCH₃ | SO₃H | H |
| (CH₂)ₘSO₂CH=CH₂ | (CH₂)ₙSO₃H | SO₃H | H |
| (CH₂) ₘSO₂CH=CH₂ | (CH₂)ₙCH₃ | SO₃H | SO₃H |
| (CH₂) ₘSO₂CH=CH₂ | (CH₂)ₙSO₃H | SO₃H | SO₃H |
| (CH₂)ₘCONHNH₂ | (CH₂)ₙCH₃ | H | H |
| (CH₂)ₘCONHNH₂ | (CH₂)ₙSO₃H | H | SO₃H |
| (CH₂)ₘCONHNH₂ | (CH₂)ₙCH₃ | SO₃H | H |
| (CH₂)ₘCONHNH₂ | (CH₂)ₙSO₃H | SO₃H | H |
| (CH₂)ₘCONHNH₂ | (CH₂)ₙCH₃ | SO₃H | SO₃H |
| (CH₂)ₘCONHNH₂ | (CH₂)ₙSO₃H | SO₃H | SO₃H |
| (CH₂)ₘCHO | (CH₂)ₙCH₃ | H | H |
| (CH₂)ₘCHO | (CH₂)ₙSO₃H | H | SO₃H |
| (CH₂)ₘCHO | (CH₂)ₙCH₃ | SO₃H | H |
| (CH₂)ₘCHO | (CH₂)ₙSO₃H | SO₃H | H |
| (CH₂) ₘCHO | (CH₂)ₙCH₃ | SO₃H | SO₃H |
| (CH₂) ₘCHO | (CH₂)ₙSO₃H | SO₃H | SO₃H |
| (CH₂) ₘY (CH₂) ₚCOOH | (CH₂)ₙCH₃ | H | H |
| (CH₂)ₘY(CH₂)ₚCOOH | (CH₂)ₙSO₃H | H | SO₃H |
| (CH₂)ₘY (CH₂)ₙCOOH | (CH₂)ₙCH₃ | SO₃H | H |
| (CH₂)ₘY(CH₂)ₚCOOH | (CH₂)ₙSO₃H | SO₃H | H |
| (CH₂)ₘY(CH₂)ₚCOOH | (CH₂)ₙCH₃ | SO₃H | SO₃H |
| (CH₂)ₘY(CH₂)ₚCOOH | (CH₂)ₙSO₃H | SO₃H | SO₃H |
| (CH₂)ₘY(CH₂)ₚCO-NHS | (CH₂)ₙCH₃ | H | H |
| (CH₂)ₘY(CH₂)ₚCO-NHS | (CH₂)ₙSO₃H | H | SO₃H |
| (CH₂)ₘY (CH₂)ₚCO-NHS | (CH₂)ₙCH₃ | SO₃H | H |
| (CH₂)ₘY (CH₂)ₚCO-NHS | (CH₂)ₙSO₃H | SO₃H | H |
| (CH₂)ₘY(CH₂)ₚCO-NHS | (CH₂) ₙCH₃ | SO₃H | SO₃H |
| (CH₂)ₘY(CH₂)ₚCO-NHS | (CH₂)ₙSO₃H | SO₃H | SO₃H |
| (CH₂)ₘY(CH₂)ₚCO-imidazole | (CH₂)ₙCH₃ | H | H |
| (CH₂)ₘY(CH₂)ₚCO-imidazole | (CH₂)ₙSO₃H | H | SO₃H |
| (CH₂)ₘY(CH₂)ₚCO-imidazole | (CH₂)ₙCH₃ | SO₃H | H |
| (CH₂)ₘY(CH₂)ₚCO-imidazole | (CH₂)ₙSO₃H | SO₃H | H |
| (CH₂)ₘY(CH₂)ₚCO-imidazole | (CH₂)ₙCH₃ | SO₃H | SO₃H |
| (CH₂)ₘY (CH₂)ₚCO-imidazole | (CH₂)ₙSO₃H | SO₃H | SO₃H |
| CH₂(CH₂)ₘO-PAM | (CH₂)ₙCH₃ | H | H |
| CH₂(CH₂)ₘO-PAM | (CH₂)ₙCH₃ | H | SO₃H |
| CH₂ (CH₂)ₘO-PAM | (CH₂)ₙCH₃ | SO₃H | H |
| CH₂ (CH₂)ₘO-PAM | (CH₂)ₙCH₃ | SO₃H | H |
| CH₂(CH₂)ₘO-PAM | (CH₂)ₙCH₃ | SO₃H | SO₃H |
| CH₂(CH₂)ₘO-PAM | (CH₂)ₙCH₃ | SO₃H | SO₃H |
| (CH₂)ₘY(CH₂)ₚO-PAM | (CH₂)ₙCH₃ | H | H |
| (CH₂)ₘY(CH₂)ₚO-PAM | (CH₂)nCH₃ | H | SO₃H |
| (CH₂)ₘYCH₂)ₚO-PAM | (CH₂)ₙCH₃ | SO₃H | H |
| (CH₂)ₘY(CH₂)ₚO-PAM | (CH₂)ₙCH₃ | SO₃H | H |
| (CH₂)ₘY(CH₂)ₚO-PAM | (CH₂)ₙCH₃ | SO₃H | SO₃H |
| (CH₂)ₘY(CH₂)ₚO-PAM | (CH₂)ₙCH₃ | SO₃H | SO₃H |

Each sulphonic moiety in the above table may be replaced by a corresponding sulphonate moiety.

Preferred symmetric cyanines according to the present invention are represented by any of the formulae (2a) to (21): 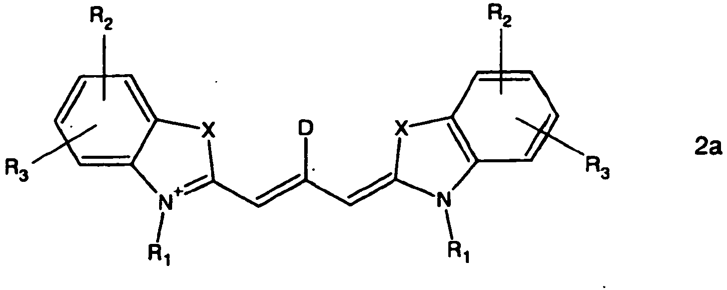 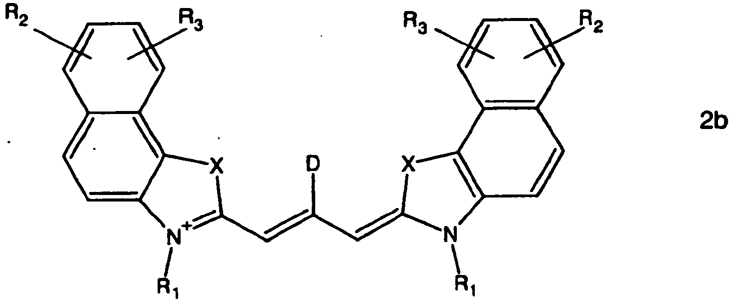 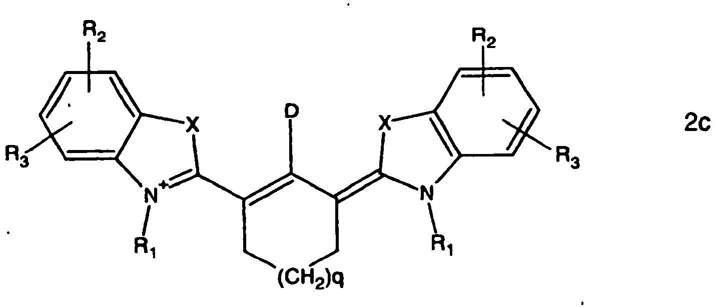 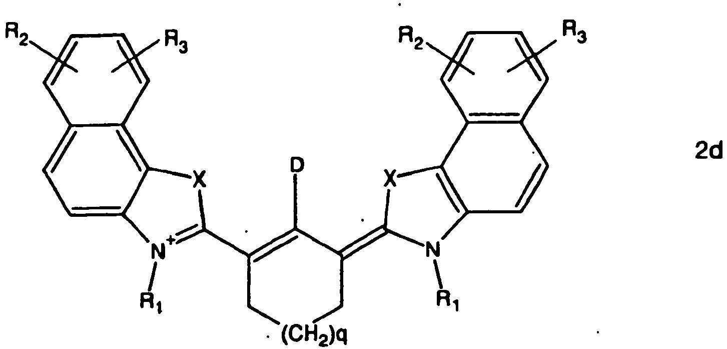 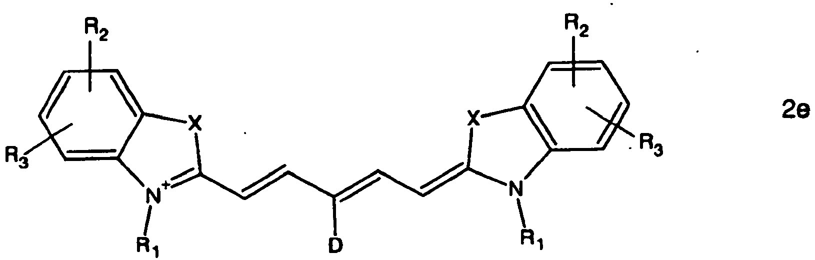 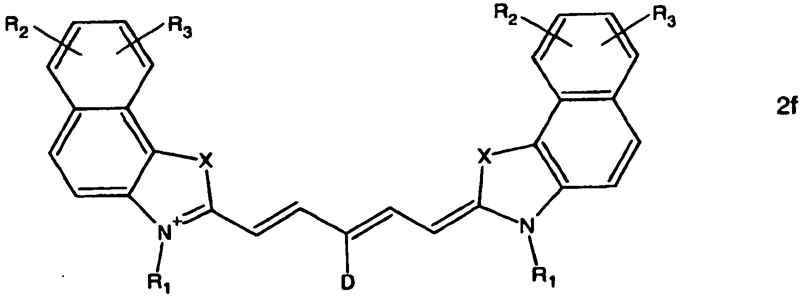 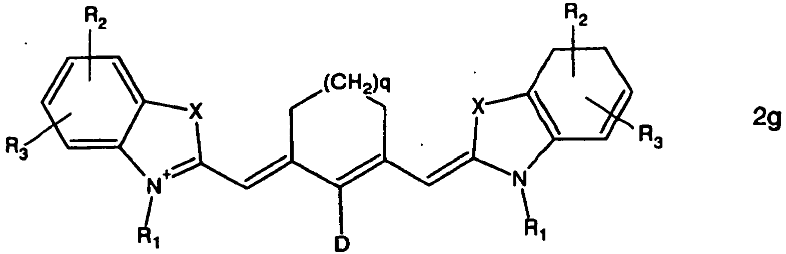 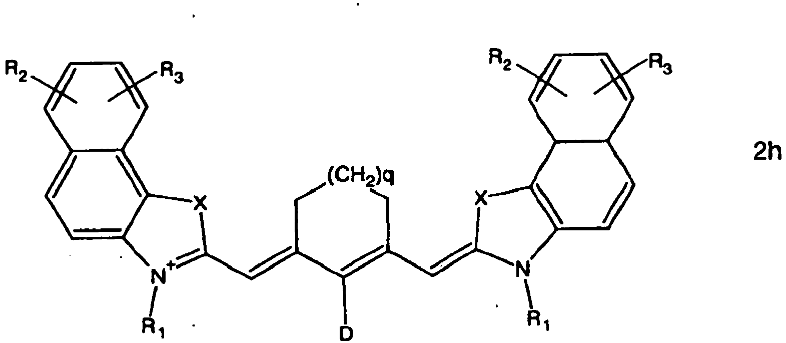 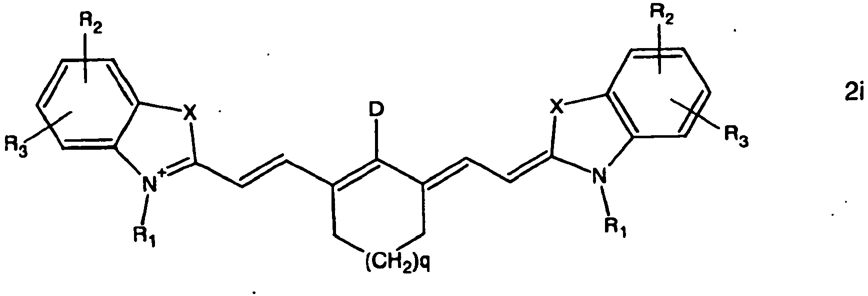 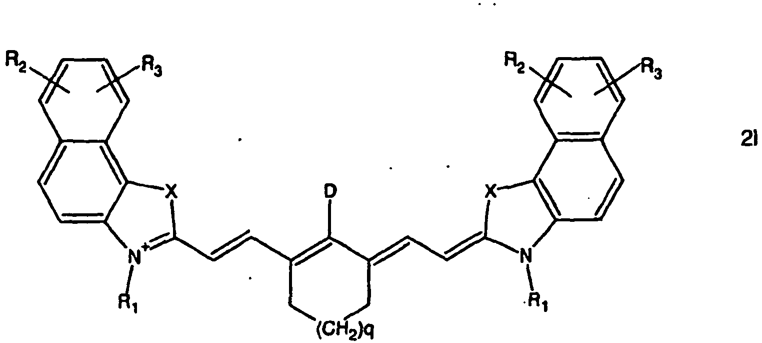 wherein R₁, R₂, R₃, X, q and D have the meanings indicated in respect of formula (1).

The following examples are simply meant to further illustrate specific applications of the present invention and are not intended to be construed as defining or limiting the scope of the invention.

### Examples

### Referential Example 1: synthesis of benzothiazolecarbo-µ-(2-carboxyethyl) cyanine iodide (Compound 1)

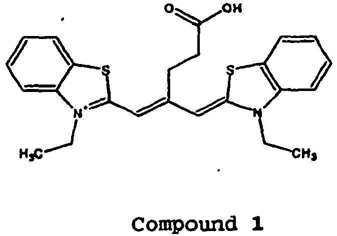

2.00 g of N-ethyl-2-methyl-benzothiazole, 5.25 g of succinic anhydride and 20 mL of pyridine are heated to reflux in a 100 mL flask for 30 minutes. After the reagents dissolve, the color of the solution turns to purple from yellow within 5 minutes. The solution is cooled to room temperature and added to a rapidly stirred solution of diethyl ether. The solid is collected on a filter funnel and then purified by flash chromatography on silica 60, 200-400 mesh, eluting with a dichloromethane/methanol 9/1 mixture. The purified product has λ_{MeOH} = 548 nm.

### Referential Example 2: synthesis of benzothiazolecarbo-µ-[3-hydroxy-(2-amidoethyl)] cyanine iodide and benzothiazolecarbo-µ-[3-amino-(2-amidoethyl)] cyanine iodide (Compounds 2a, 2b)

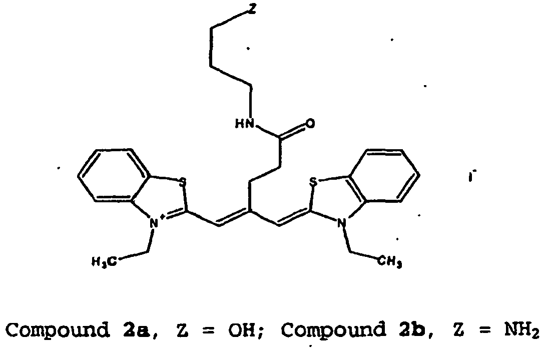

Compound **1** is dissolved in 1 mL of anhydrous N,N-dimethylformamide and equimolar amounts of dicyclohexylcarbodiimide and 1-hydroxybenzotriazole hydrate are added. After stirring for 5 minutes, a 5 fold excess of 1,3-aminopropanol is added. The reaction mixture is stirred overnight and then is added dropwise to 50 mL of rapidly stirred ether. The product is washed with ether and dried in a desiccator. 0.40 g of a fuchsia solid (compound **2a**) are thus obtained, with λ_{MeOH} = 550nm. An amino-functionalised dye (compound **2b)** is obtained by using an excess of 1,3-diaminopropane in place of 1,3-aminopropanol.

### Referential Example 3: synthesis of benzothiazolecarbo-µ-[3-phosphorami-dite-(2-amidoethyl)]cyanine iodide (Compound 3)

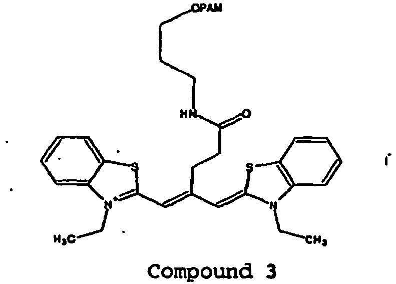

0.50 g of alcohol **2a** is dried in a vacuum oven at 40°C for five hours and then is loaded into a dry, 100 mL, 3-neck flask. 40 mL of anhydrous acetonitrile are added under argon, followed by 0.17 mL of a 0.5 M solution of tetrazole in acetonitrile and 0.42 mL of 2-cyanoethyltetraisopropylphos-phorodiamidite. The solution is stirred under argon for 90 minutes at room temperature. After this time period it is evaporated in vacuo. The residue is re-dissolved in 1 mL of acetonitrile and precipitated by dropwise addition to 100 mL of anhydrous ether. It is stored at -20°C. The yield of **3** is 98%.

### Referential Example 4: synthesis of n,n-bis(sulfobutyl)benzothiazole-benzothiazolecarbo-µ-(2-carboxyethyl) cyanine sodium salt (Compound 4)

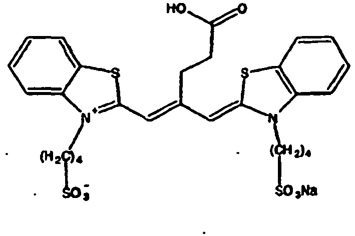

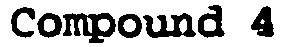

2.00 g of N-(δ-sulfonatobutyl)-2-methyl-benzothiazole, 5.25 g of succinic anhydride and 20 mL of pyridine are heated to reflux in a 100 mL flask for 30 minutes. After the reagent dissolve, the color of the solution turns to purple from yellow within 5 minutes.. The solution is cooled to room temperature and added to a rapidly stirred solution of diethyl ether. The solid is collected on a filter funnel and then purified by reverse phase medium pressure chromatography on RP-18 LichroPrep (Merck), 25-40 µm. The product is eluted with a methanol/water 60/40 solvent mixture. The purified product has λ_{MeOH} = 550 nm.

### Referential Example 5: synthesis of n,n-bis(sulfobutyl)benzothiazole-carbo-µ-[4-carboxybutyl-(2-amidoethyl)] cyanine sodium salt (Compound 5)

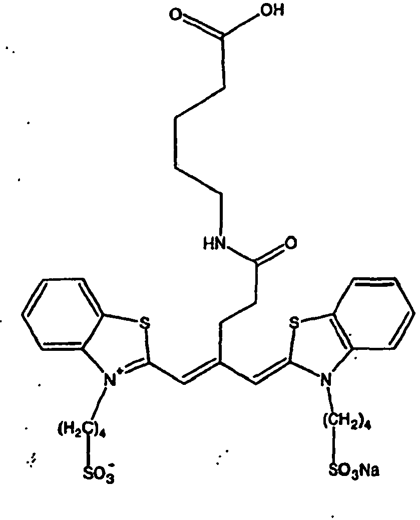

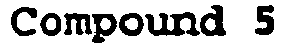

0.50 g of compound **4** is dissolved in 1 mL of anhydrous N,N-dimethylformamide and equimolar amounts of dicyclohexylcarbodiimide and 1-hydroxybenzotriazole hydrate are added. After stirring for 5 minutes, a 2 fold excess of 4-aminobutyric acid t-butyl ester is added. The reaction mixture is stirred overnight and then is added dropwise to 50 mL of rapidly stirred ether. The product is washed with ether and dried in a desiccator. The crude t-butyl ester is saponified by treatment with 1 mL of trifluoroacetic acid at room temperature. After 1 hour the trifluoroacetic acid is removed in vacuo to give a fuchsia solid. The crude product was purified by purified by reverse phase medium pressure chromatography on RP-18 LichroPrep (Merck), 25-40 µm. The product is eluted with a methanol/water 70/30 solvent mixture. The purified product, compound 5, has λ_{MeOH} = 550 nm.

### Referential Example 6: synthesis of n,n-bis(sulfobutyl)benzothiazolecar-bo-µ-[4-succinimidyl ester butyl-(2-amidoethyl)] cyanine sodium salt (Compound 6)

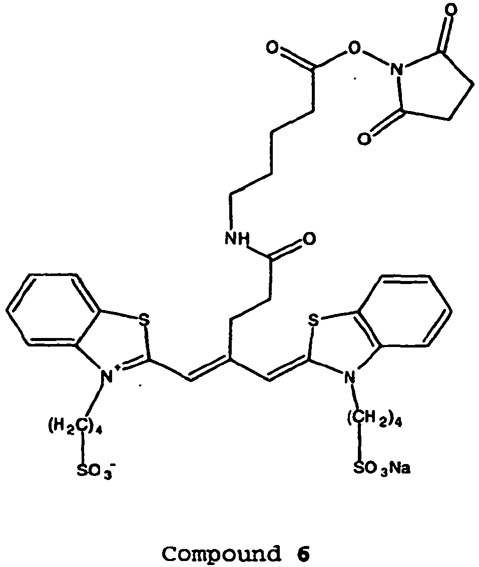

The acid **5** is converted to its N-hydroxysuccinimide ester as follows. 100 mg of the acid, and equimolar amounts of N.N'-dicyclohexylcarbodiimide and N-hydroxysuccinimide are dissolved in 3 mL of dry DMF in a microsynthesis vial. All glassware and reagents must be rigorously anhydrous. The solution is stirred overnight at 50°C. The active ester **6** is precipitated with anhydrous ether, collected on a glass filter and washed five times with anhydrous ether, dried and stored at -20°C. Yield of ester from the acid was 90%.

### Example 7: synthesis of bromomalonaldehyde dianil bromide (Compound 7)

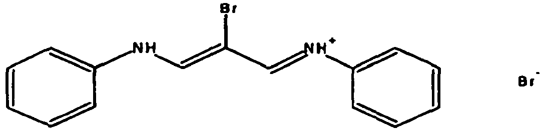

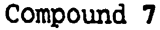

3.54 g of aniline are dissolved in 15 mL of ethanol in a 100 mL beaker. Separately, 5 g of muchobromic acid are dissolved in 15 mL of ethanol in a 100 mL Erlenmeyer flask. This solution is added drop by drop to the aniline/ethanol solution, with cooling. The reaction mixture turns immediately yellow, then orange, with development of CO₂. At the end of the addition, the mixture is heated in a water bath until its volume is reduced to one half. The resulting solution is cooled with an ice-salt mixture. A yellow crystalline mass is formed. This is collected on a fritted glass filter. A first fraction of 3.84 of pure product is obtained, 52% yield. From the mother solution a further 2.7 g of somewhat less pure product is recovered, which can be recrystallised from a small amount of ethanol to yield a further 1.7 g of pure product.

### Example 8: synthesis of sulfoindodicarbo-µ-(bromo) cyanine iodide (Compound 8)

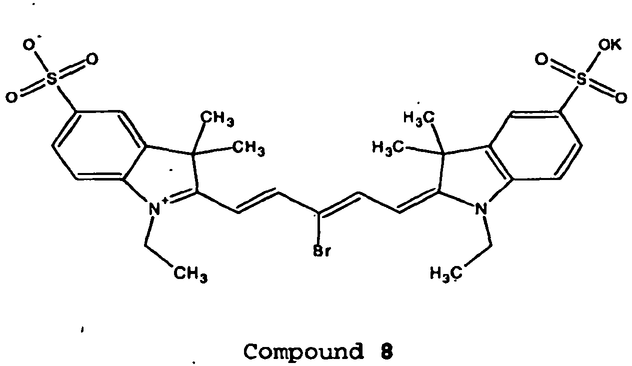

4 g of N-ethyl-2,3,3-trimethyl-3[H]indolium 5-sulphonate, 2.86 g of compound **7**, 1.87 g of pyridine and 40 mL of acetic anhydride in a 100 mL flask are heated at reflux with stirring for 2 hours. The blue solution is cooled to room temperature and added dropwise to 400 mL of rapidly stirred diethyl ether. The blue-greenish solid is collected on a fritted glass filter, washed with ether and dried in a desiccator. The crude product is purified by reverse phase medium pressure chromatography on RP-18 LichroPrep (Merck), 25-40 µm. The product is eluted with a methanol/water 70/30 solvent mixture. Yield: 65%. The purified product has λ_{MeOH} = 651 nm.

### Example 9: synthesis of sulfoindodicarbo-µ-(4-carboxy-1-buti-nyl) cyanine iodide (Compound 9)

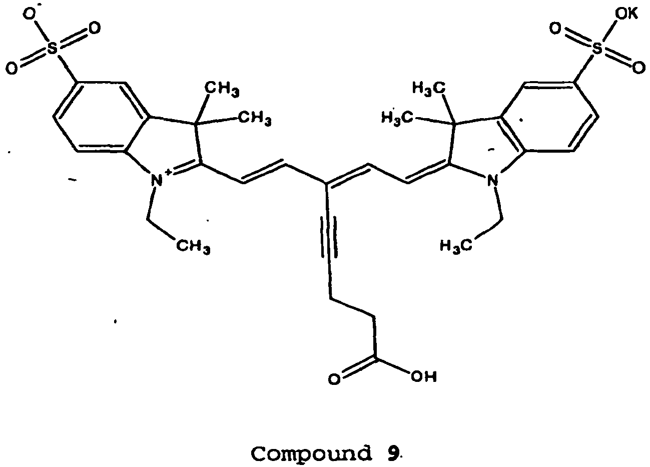

2 g of compound **8**, 300 mg of 4-pentynoic acid and 1 mL of pyrrolidine are stirred into 15 mL of N,N-dimethylformamide under nitrogen at room temperature. 200 mg of bis(triphenylphosphine)-palladium(II) chloride, and 50 mg of cooper(I) iodide are added to the reaction mixture. After 4 hours, the solvent and volatile compounds are evaporated under vacuum. The crude product is purified by reverse phase medium pressure chromatography on RP-18 LichroPrep (Merck), 25-40 µm. The product is eluted with a methanol/water 70/30 solvent mixture. Yield: 65%. The purified product has λ_{MeOH} = 655 nm

### Example 10: synthesis of sulfoindodicarbo-µ-(4-succinimidyl ester -1-butinyl) cyanine potassium salt (Compound 10)

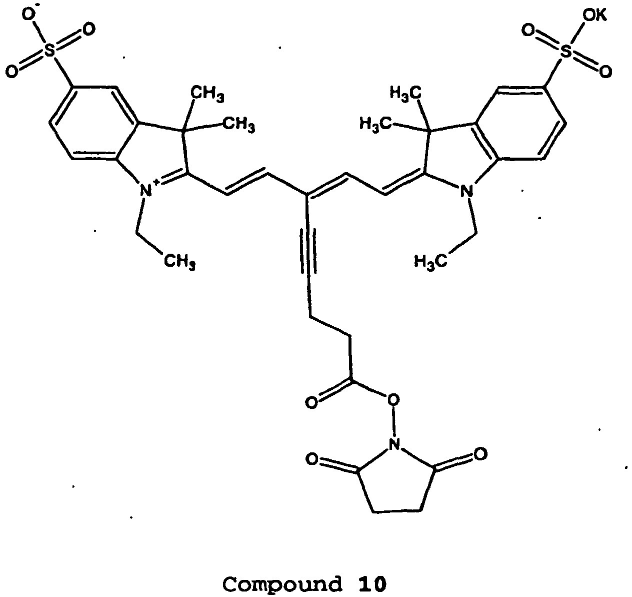

The acid **9** is converted to its N-hydroxysuccinimide ester as follows. 300 mg of the acid, and equimolar amounts of N.N'-dicyclohexylcarbodiimide and N-hydroxysuccinimide are dissolved in 10 mL of dry DMF in a microsynthesis vial. All glassware and reagents must be rigorously anhydrous. The solution is stirred overnight at 50°C. The active ester **10** is precipitated with anhydrous ether, collected on a glass filter and washed five times with anhydrous ether, dried and stored at -20°C. Yield of ester from the acid was 90%.

### Example 11: synthesis of indodicarbo-µ-(bromo) cyanine iodide (Compound 11)

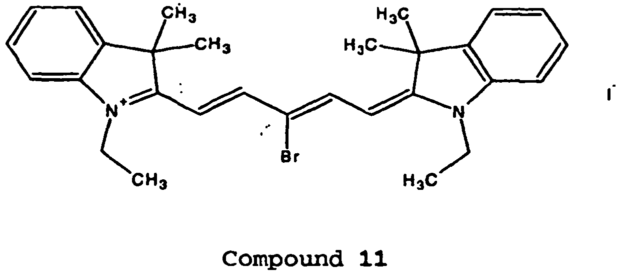

4.4 g of N-ethyl-2,3,3-trimethyl-3[H]indolium iodide, 2.86 g of compound **7**, 1.87 g of pyridine and 40 mL of acetic anhydride in a 100 mL flask are heated at reflux with stirring for 2 hours. The blue solution is cooled to room temperature and added dropwise to 400 mL of rapidly stirred diethyl ether. The blue solid is collected on a synthered glass filter, washed with ether and deride in a desiccator. The product was purified by flash chromatography on silica 60, 200-400 mesh, eluting with a dichloromethane/methanol 95/5 mixture The purified product has λ_{MeOH} = 644 nm

### Example 12: synthesis of indodicarbo-µ-(4-carboxy-1-butinyl) cyanine iodide (Compound 12)

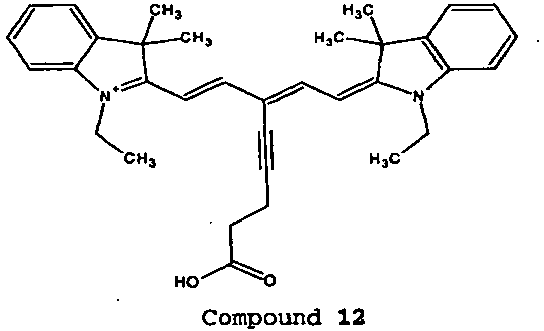

2 g of compound **11**, 300 mg of 4-pentynoic acid and 1 mL of pyrrolidine are stirred into 15 mL of N,N-dimethylformamide under nitrogen at room temperature. 200 mg of bis(triphenylphosphine)-palladium(II) chloride, and 50 mg of cooper(I) iodide are added to the reaction mixture. After 4 hours, the solvent and volatile compounds are evaporated under vacuum. The crude product is purified by flash chromatography on silica 60, 200-400 mesh, eluting with a dichloromethane/methanol 9/1 mixture. Yield: 90%. The purified product has λ_{MeOH} = 645 nm.

### Example 13: synthesis of indodicarbo-µ-[4-(3-hydroxypropyl-amido)-1-butinyl] cyanine iodide and indodicarbo-µ-[4-(3-ami-nopropylamido)-1-butinyl] cyanine iodide (Compounds 13a, 13b)

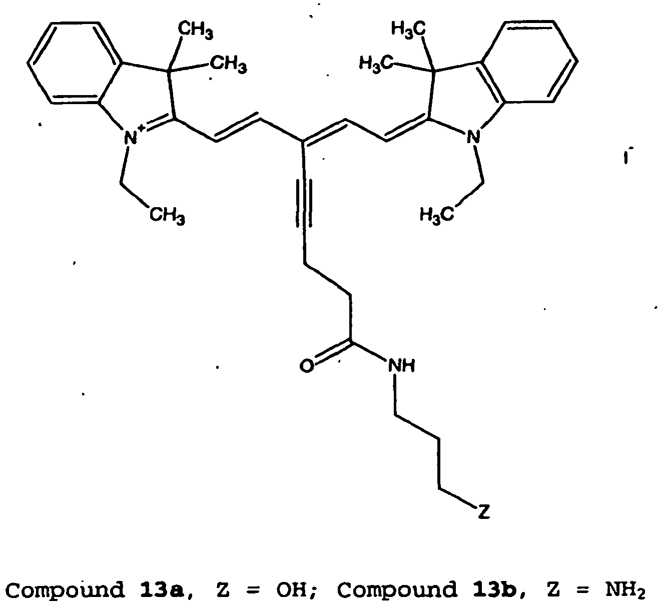

0.50 g of compound **12** is dissolved in 1 mL of anhydrous N,N-dimethylformamide and equimolar amounts of dicyclohexylcarbodiimide and 1-hydroxybenzotriazole hydrate are added. After stirring for 5 minutes, a 5 fold excess of 1,3-aminopropanol is added. The reaction mixture is stirred overnight and then is added dropwise to 50 mL of rapidly stirred ether. The product is washed with ether and dried in a desiccator. 0.40 g of a blue solid (compound **13a)** are thus obtained, with λ_{MeOH} = 648nm. An amino-functionalised dye (compound **13b)** is obtained by using an excess of 1,3-diaminopropane in place of 1,3-aminopropanol.

### Example 14: synthesis of indodicarbo-µ-[4-(3-phopsphoramidite propylamido)-1-butinyl] cyanine iodide (Compound 14)

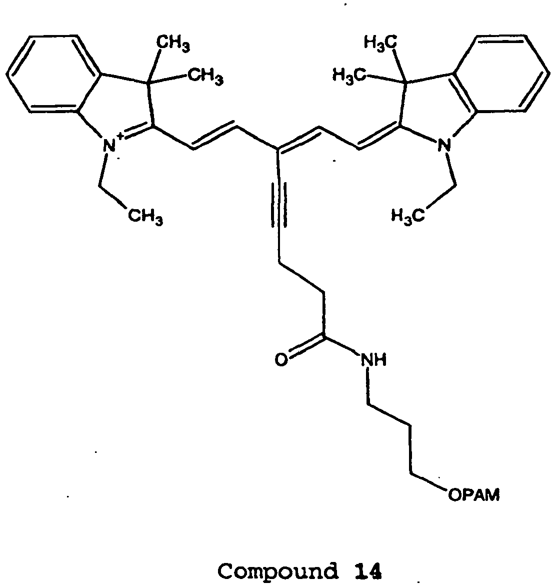

0.50 g of alcohol **13** is dried in a vacuum oven at 40°C for five hours and then is loaded into a dry, 100 mL, 3-neck flask. 40 mL of anhydrous acetonitrile are added under argon, followed by 0.17 mL of a 0.5 M solution of tetrazole in acetonitrile and 0.42 mL of 2-cyanoethyltetraisopropylphos-phorodiamidite. The solution is stirred under argon for 90 minutes at room temperature. After this time period it is evaporated in vacuo. The residue is re-dissolved in 1 mL of acetonitrile and precipitated by dropwise addition to 100 mL of anhydrous ether. It is stored at -20°C. The yield of **14** is 95%.

### Example 15: synthesis of p-carboxyphenylmalonaldehyde dianil chloride (Compound 15)

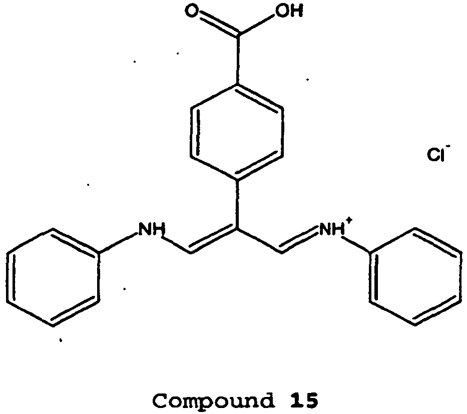

28 mL of POCl₃ are added to 39 mL stirred, cooled N,N-dimethylformamide, followed by 16 g of p-cyanophenylacetic. After 1 hour, the reaction mixture is heated at 80-90°C until carbon dioxide is no longer evolved, about 6 hours. The mixture is cooled to room temperature, mixed within 100 g of ice, and the aqueous mixture is shaken with a small amount of charcoal. The aqueous solution is made basic with potassium carbonate and extracted 3 times with 200 mL portions of dichloromethane. The combined organic layers are washed with distilled water, dried with sodium sulphate and evaporated to a dark oil. This oil is subjected to basic hydrolysis to produce the free dihaldehyde and at the same time convert the nitrile to the corresponding carboxylate. Thus, the oil is suspended in 100 mL of water and 25 mL of 50% aqueous NaOH are added with stirring. The mixture is heated at 70°C until a homogenous aqueous solution results. The solution is neutralised with concentrated hydrochloric acid and 25 g of aniline hydrochloride dissolved in 100 mL of water are added. The yellow orange precipitate is collected on a fritted glass filter and dried in the oven at 50°C.

### Example 16: synthesis of indodicarbo-µ-(4-carboxyphenyl) cyanine iodide (Compound 16)

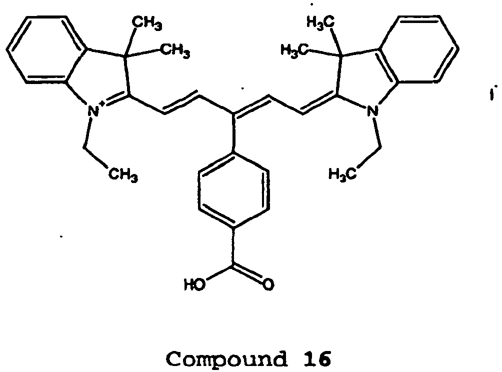

4.4 g of N-ethyl-2,3,3-trimethyl-3[H]indolium iodide, 2.64 g of compound **15**, 2.00 g of pyridine and 50 mL of acetic anhydride in a 100 mL flask are heated at reflux with stirring for 2 hours. The blue solution is cooled to room temperature and added dropwise to 400 mL of rapidly stirred diethyl ether. The blue solid is collected on a fritted glass filter, washed with ether and dried in a desiccator. The product was purified by flash chromatography on silica 60, 200-400 mesh, eluting with a dichloromethane/methanol 95/5 mixture. The purified product has λ_{MeOH} = 648 nm.

### Example 17: synthesis of indodicarbo-µ-[4-(3-hydroxypropyl-amido)phenyl] cyanine iodide and indodicarbo-µ-[4-(3-amino-propylamido)-1-butinyl] cyanine iodide (Compounds 17a, 17b)

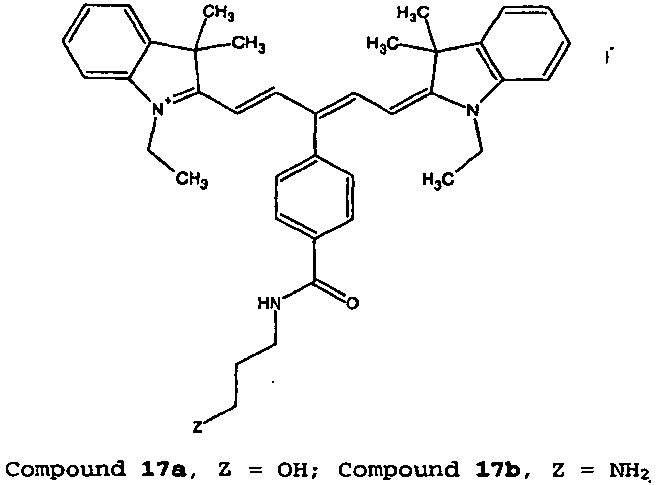

0.50 g of compound **16** is dissolved in 1 mL of anhydrous N,N-dimethylformamide and equimolar amounts of dicyclohexylcarbodiimide and 1-hydroxybenzotriazole hydrate are added. After stirring for 5 minutes, a 5 fold excess of 1,3-aminopropanol is added. The reaction mixture is stirred overnight and then is added dropwise to 50 mL of rapidly stirred ether. The product is washed with ether and dried in a desiccator. 0.40 g of a blue solid (compound **17a)** are thus obtained, with λ_{MeOH} = 647nm. An amino-functionalised dye (compound **17b)** is obtained by using an excess of 1,3-diaminopropane in place of 1,3-aminopropanol.

### Example 18: synthesis of chloromalonaldehyde dianil chloride (Compound 18)

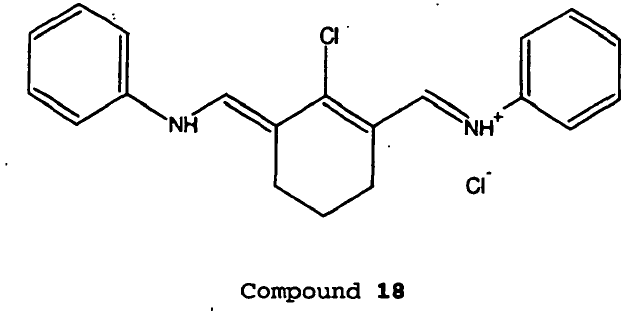

13 mL of anhydrous DMF are added under argon to a 250 mL flask fitted with a mechanical stirrer. 11 ml of POCl₃, previously cooled to 4°C in the refrigerator, are slowly added drop by drop. The reaction mixture is cooled to 0°C with a NaCl/ice bath under continuous stirring. A solution of 2.65 mL of cyclohexanone in 5 mL of dichloromethane is added dropwise. The color of the reaction mixture turn to yellow. At the of the addition, the mixture is stirred for a further 15 minutes and then heated on a water bath for 1 hour. It is then cooled to room temperature and a cold solution of 10 mL of aniline in 10 mL of ethanol is added dropwise. The reaction mixture turn to deep violet and becomes very viscous. 100 mL of cold water and 10 mL of cold concentrated HCl are added. The reaction mixture is transferred to a beaker, covered and kept in a refrigerator at 4°C overnight. A dark violet crystalline mass precipitates and is collected on a fritted glass filter and washed several times with cold water. The product is dried overnight in a desiccator. The UV-Vis absorption spectrum shows two peaks, at 520 and 415 nm.

### Example 19: synthesis of 4-(2-carboxyethylamido)phenol, 4-(2-carboxypropylamido)phenol, 4-(2-carboxyethylamido)thiophenol and 4-(2-carboxypropylamido)thiophenol (Compounds 19a, 19b, 19c, 19d)

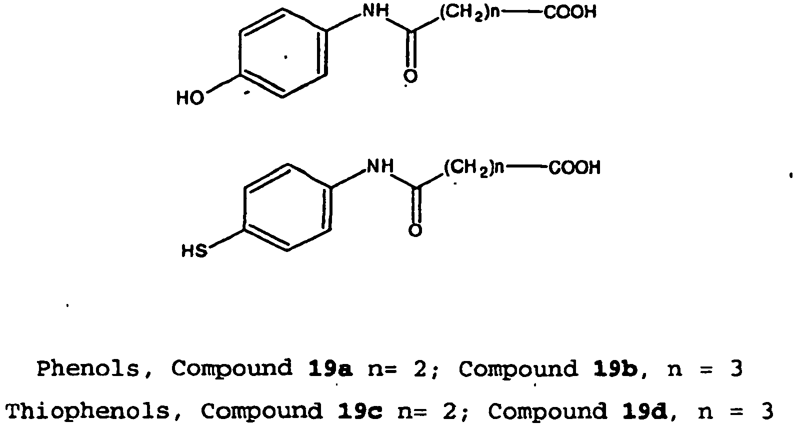

11 g of p-aminophenol are suspended in 100 ml of water in a 500 mL flask. A suspension of 14 g of succinic anhydride in 100 mL of water is added with stirring. The mixture is warmed to 50°C. A crystalline mass precipitates. It is dissolved again by heating to the boil. The solution is cooled to room temperature. A white crystalline mass forms. This is collected on a fritted glass filter and washed with two portions of 50 mL of cold water and is dried in air. The yield of **19a** is 86%. Compound **19b** was similarly prepared from 11 g of p-aminophenol and 15 g of glutaric anhydride, yield 95%. The corresponding thiophenols (compounds **19c** and **19d**) were similarly prepared from 4-mercaptoaniline and succinic and glutaric anhydrides, respectively.

### Example 20: synthesis of indotricarbocyclohexen-µ-(chloro) cyanine iodide (Compound 20)

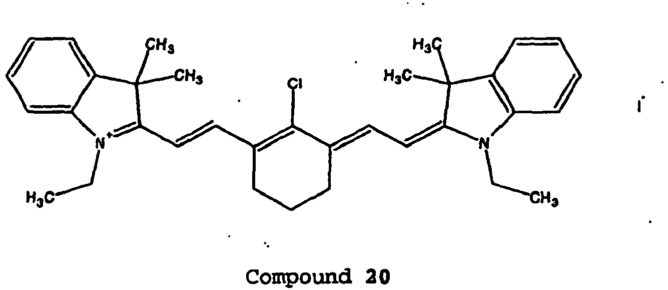

20 g of N-ethyl-2,3,3-trimethyl-3[H]indolium iodide, 11.4 g of compound **18**, 6. 3 g of sodium acetate anhydrous and 400 mL of ethanol are refluxed in a 1000 mL flask for 1 hour. The solution is cooled to room temperature and slowly added to 4 L of diethyl ether. The green precipitate is collected on a fritted glass filter and purified by flash chromatography on silica 60, 200-400 mesh, eluting with a dichloromethane/methanol 9/1 mixture.

### Referential Example 21: synthesis of indotricarbocyclohexen-µ-[3-(3-hy-droxypropylamido)propylamidothiophenoxy] cyanine iodide and indotricarbocyclohexen-µ-[3-(3-aminopropylamido)propylamido-thiophenoxy] cyanine iodide (Compounds 21a, 21b)

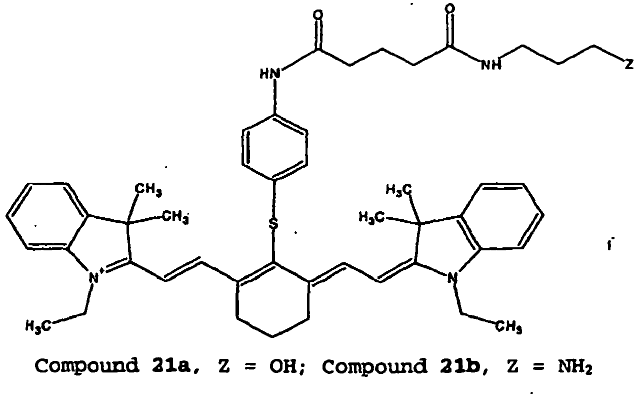

All the glassware is dried overnight at 120°C. Compounds **20** and **19d** are dried in a vacuum oven for 90 minutes at 40°C over silica gel. A 3-neck, 100 mL flask, cooled under a stream of nitrogen is loaded with 0.5 g of compound **20** and 1.72 g of compound **19d**. 10 mL of anhydrous N,N-dimethylformamide are added by cannula under nitrogen. The mixture is stirred for 15 minutes under nitrogen. The solvent is evaporated and the residue dissolved in a small amount of methanol. The green methanol solution is filtered and added to 200 mL of rapidly stirred ether. The green precipitate is collected on a sintered glass filter and purified by purified by reverse phase medium pressure chromatography on RP-18 LichroPrep (Merck), 25-40 µm. The product is eluted with a methanol/water 70/30 solvent mixture. 0.40 g of an emerald green solid are thus obtained, with λ_{MeOH} = 785 nm. The cyanine acid is dissolved in 1 mL of anhydrous N,N-dimethylformamide and equimolar amounts of dicyclohexylcarbodiimide and 1-hydroxybenzotriazole hydrate are added. After stirring for 5 minutes, a 5 fold excess of 1,3-aminopropanol is added. The reaction mixture is stirred overnight and then is added dropwise to 50 mL of rapidly stirred ether. The product is washed with ether and dried in a desiccator. 0.40 g of an emerald green solid (compound **21a**) are thus obtained, with λ_{MeOH} = 785. An amino-functionalised dye (compound **21b**) is obtained by using an excess of 1,3-diaminopropane in place of 1,3-aminopropanol.

### Referential Example 22: synthesis of indotricarbocyclohexen-µ-[3-(3-phopsphoramidite propylamido) propylamido thiophenoxy] cyanine iodide (Compound 22)

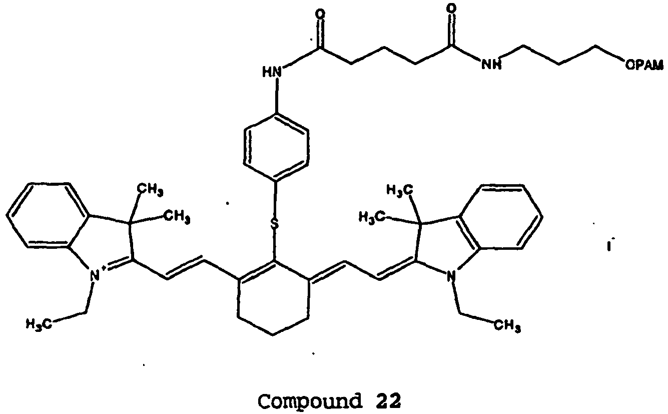

0.50 g of alcohol **21a** is dried in a vacuum oven at 40°C for five hours and then is loaded into a dry, 100 mL, 3-neck flask. 40 mL of anhydrous acetonitrile are added under argon, followed by 0.17 mL of a 0.5 M solution of tetrazole in acetonitrile and 0.42 mL of 2-cyanoethyltetraisopropylphos-phorodiamidite. The solution is stirred under argon for 90 minutes at room temperature. After this time period it is evaporated in vacuo. The residue is re-dissolved in 1 mL of acetonitrile and precipitated by dropwise addition to 100 mL of anhydrous ether. It is stored at -20°C. The yield of **22** is 90%.

### Referential Example 23: synthesis of sulfoindotricarbocyclohexen-µ-(chloro) cyanine (Compound 23)

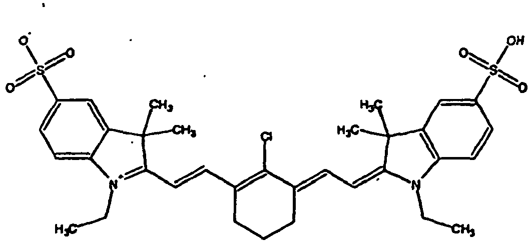

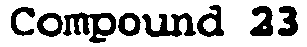

20 g of N-ethyl-2,3,3-trimethyl-3[H]-indolium-5-sulfonate, 13.5 g of compound **18,** 6.10 g of sodium acetate anhydrous and 400 mL of ethanol are refluxed for 1 hour under stirring in a 1000 mL flask. The solution is cooled to room temperature and slowly added to 4 L of rapidly stirred diethyl ether. The green solid is collected on a filter and purified by reverse phase medium pressure chromatography on RP-18 LichroPrep (Merck), 25-40 µm. The product was eluted with a methanol/water 60/40 solvent mixture.

### Referential Example 24: synthesis of sulfoindotricarbocyclohexen-µ-[(3-carboxypropyl)amido]thiophenoxy cyanine sodium salt (Compound 24)

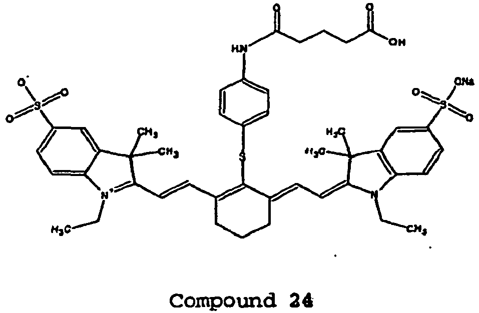

All the glassware is dried overnight at 120°C. Compounds **23** and **19d** are dried in a vacuum oven for 90 minutes at 40°C over silica gel. A 3-neck, 100 mL flask, cooled under a stream of nitrogen is loaded with 0.5 g of compound **23** and 1.72 g of compound **19d**. 10 mL of anhydrous N,N-dimethylformamide are added by cannula under nitrogen. The mixture is stirred for 15 minutes under nitrogen. The solvent is evaporated and the residue dissolved in a small amount of methanol. The green methanol solution is filtered and added to 200 mL of rapidly stirred ether. The green precipitate is collected on a sintered glass filter and purified by purified by reverse phase medium pressure chromatography on RP-18 LichroPrep (Merck), 25-40 µm. The product is eluted with a methanol/water 70/30 solvent mixture. 0.55 g of a green solid are thus obtained, with λ_{MeOH} = 794 nm

### Referential Example 25: synthesis of sulfoindotricarbocyclohexen-µ-[(3-succinimidyl ester propyl)amido]thiophenoxy cyanine sodium salt (Compound 25)

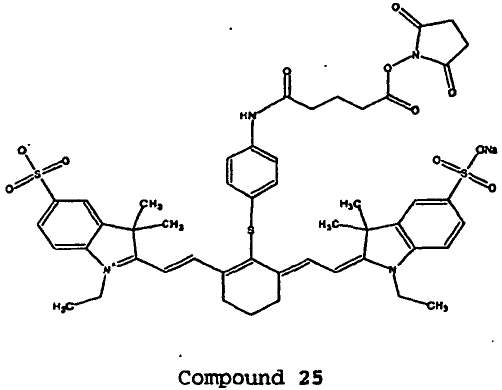

The acid 23 is converted to its N-hydroxysuccinimide ester as follows. 100 mg of the acid **24,** and equimolar amounts of N.N'-dicyclohexylcarnodiimide and N-hydroxysuccinimide are dissolved in 3 mL of dry DMF in a microsynthesis vial. All glassware and reagents must be rigorously anhydrous. The solution is stirred overnight at 50°C. The active ester **25** is precipitated with anhydrous ether, collected on a glass filter and washed five times with anhydrous ether, dried and stored at -20°C. Yield of ester from the acid was 95%.

### Referential Example 26: synthesis of the conjugate between sulfoindotricarbocyclohexen-µ-[(3-succinimidyl ester propyl)amido]thiophenoxy cyanine sodium salt and 5-allylamino-dUTP (Compound 26)

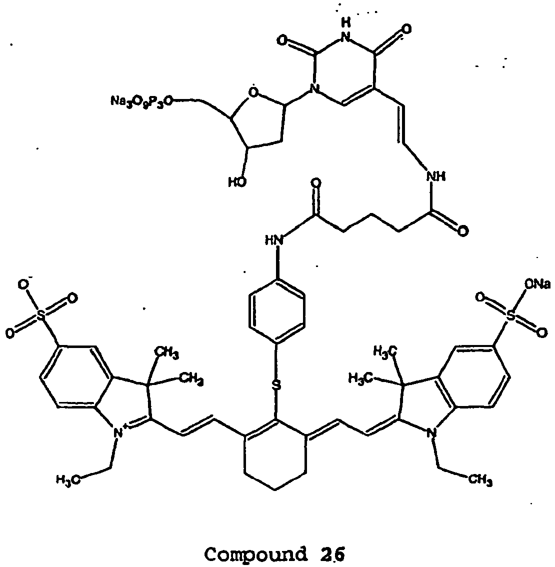

2 µmol of 5-allylamino-dUTP is dissolved in 1.2 mL 0.1 M borate buffer pH 8. 10 µmol of active ester **25** dissolved in 300 µmol of DMF are added to the 5-allylamino-dUTP solution and the mixture is stirred in the dark at room temperature. The reaction is monitored by RP-HPLC (column: Waters Novapack 3.9x150 mm; loop: 20 mL; flow rate: 1 mL/min; program: 15' linear gradient from 100% A to 50% A /50%B, 5' 50%A/50% B, 5' gradient back to 100 A%, with A = water with 0.1% trifluoroacetic acid and B = acetonitrile. The crude conjugate solution is prepurified by gel filtration chromatography on a 1.5x30 cm, Sephadex G-10 column, with water as eluent. The final purification is by medium pressure liquid chromatography on a Lichroprep RP-18, 20x300 column, with water/acetonitrile 70:30 as eluent. The coupling efficiency was 85% . The conjugate is stored at -20°C.

### Referential Example 27: synthesis of 2-formyl-5-hydroxylyden-1-chlorocy-clopenten (Compound 27)

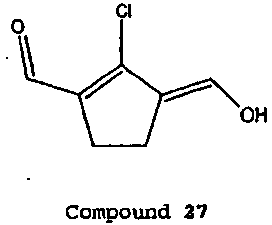

10 mL of anhydrous DMF and 10 mL of dichloromethane are added under argon to a 250 mL flask fitted with a mechanical stirrer. The mixture is cooled with a water/NaCl bath to 4-5°C. A solution of 9 ml of POCl₃ in 9 mL of dichloromethane previously cooled to 4°C in the refrigerator, is slowly added drop by drop. The reaction mixture is cooled to 0°C with a NaCl/ice bath under continuous stirring. The addition requires 45 minutes. The reaction mixture becomes milky and it is allowed to stand for 30 minutes at 4-5°C. A solution of 2.00 mL of cyclopentanone is added dropwise. The color of the reaction mixture turns to yellow. At the of the addition, the mixture is stirred for a further 15 minutes and then heated at reflux for 5 hours. Its color turns to orange and then to dark red. It is then cooled to room temperature. The dichloromethane solvent is evaporated in vacuo and the residue is thrown into 100 g of ice and is stirred for 2 hours. The pH of the solution is brought to 5 with 50% aqueous NaOH. A dark precipitate forms. The mixture is stirred overnight and the violet crystals are collected onto a fritted glass filter. Yield 2.46 g, 67%. The product, dissolved in methanol has a peak at 337 nm.

### Referential Example 28: synthesis of benzo[e]indotricarbocyclopenten-1-(chloro) cyanine iodide (Compound 28)

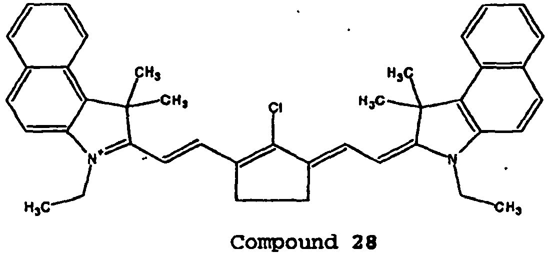

1.0 g of compound 3-ethyl-1,1,2-trimethyl-benz[e]-1[H]indo-lium iodide, 0.20 g of compound **27**, 3 g of sodium acetate anhydrous and 10 mL are heated at reflux in a 3-neck, 50 mL flask for 30 minutes. The solution color turns to dark violet. The mixture is cooled to room temperature and is added dropwise to 500 mL of rapidly stirred ether and purified by flash chromatography on silica 60 200-400 mesh, eluting with a dichloromethane/methanol 9/1 mixture. Yield, 1.10 g. λ_{MeOH} = 839 nm.

### Referential Example 29: synthesis of benzo[elindotricarbocyclopenten-µ-[3-(3-hydroxypropylamido)propylamido] thiophenoxy cyanine iodide and benzo[e]indotricarbocyclopenten-µ-[3-(3-aminopropylamido)propylamido] thiophenoxy cyanine iodide (Compounds 29a, 29b)

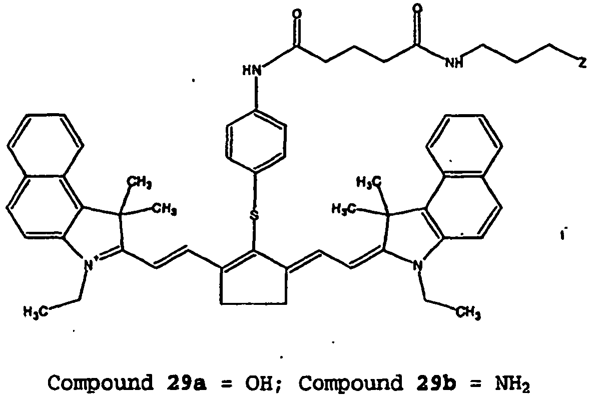

All the glassware is dried overnight at 120°C. Compounds **28** and **19d** are dried in a vacuum oven for 90 minutes at 40°C over silica gel. A 3-neck, 100 mL flask, cooled under a stream of nitrogen is loaded with 0.5 g of compound **28** and 2.0 g of compound **19d** mL of anhydrous N,N-dimethylformamide are added by cannula under nitrogen. The mixture is stirred for 15 minutes under nitrogen. The solvent is evaporated and the residue dissolved in a small amount of methanol. The green methanol solution is filtered and added to 200 mL of rapidly stirred ether. The green precipitate is collected on a fritted glass filter and purified by purified by reverse phase medium pressure chromatography on RP-18 LichroPrep (Merck), 25-40 µm. The product is eluted with a methanol/water 70/30 solvent mixture. 0.40 g of an emerald green solid are thus obtained, with λ_{MeOH} = 785 nm. The cyanine acid is dissolved in 1 mL of anhydrous N,N-dimethylformamide and equimolar amounts of dicyclohexylcrbodiimide and 1-hydroxybenzotriazole hydrate are added. After stirring for 5 minutes, a 5 fold excess of 1,3-aminopropanol is added. The reaction mixture is stirred overnight and then is added dropwise to 50 mL of rapidly stirred ether. The product is washed with ether and dried in a desiccator. 0.40 g of an emerald green solid (compound **29a)** are thus obtained, with λ_{MeoH} = 850nm. An amino-functionalised dye **(29b)** compound is obtained by using an excess of 1,3-diaminopropane in place of 1,3-aminopropanol.

### Referential Example 30: synthesis of benzo[e]indotricarbocyclopenten-µ-[3-(3-phosphoramidite propylamido)propylamido] thiophenoxy cyanine iodide (Compound 30)

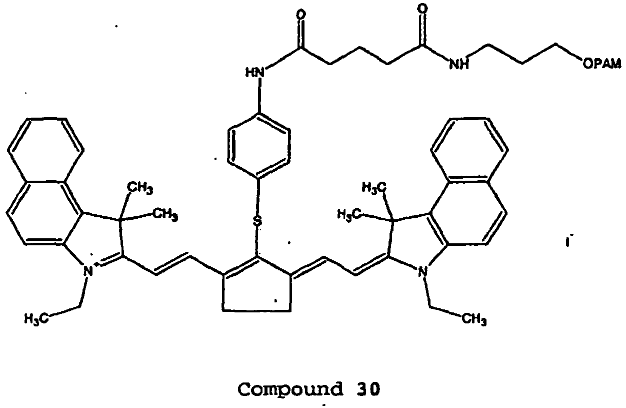

0.50 g of alcohol **29a** is dried in a vacuum oven at 40°C for five hours and then is loaded into a dry, 100 mL, 3-neck flask. 40 mL of anhydrous acetonitrile are added under argon, followed by 0.17 mL of a 0.5 M solution of tetrazole in acetonitrile and 0.42 mL of 2-cyanoethyltetraisopropylphos-phorodiamidite. The solution is stirred under argon for 90 minutes at room temperature. After this time period it is evaporated in vacuo. The residue is re-dissolved in 1 mL of acetonitrile and precipitated by dropwise addition to 100 mL of anhydrous ether. It is stored at -20°C. The yield of **30** is 60%.

### Example 31: synthesis of 2-(4-pyridyl) malondialdehyde (Compound 31)

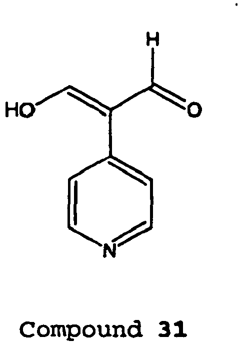

To 124 ml of N,N-dimethylformamide (DMF), stirred and cooled at +5°C, are added under argon 29.6 ml of POCl₃. The temperature is carried to room temperature and the mixture is stirred at this temperature for half an hour, then is cooled to -10°C and 10.16 ml of picoline are added. The mixture is heated at 70°C for 6 hours, then is cooled over night at room temperature and then mixed within 376 g of ice, stirred until the ice is melting.

The aqueous solution is subjected to basic hydrolysis to produce the free dihaldehyde. An aqueous solution of NaOH (64.4 g in 107 ml of water) is added dropwise. The mixture is stirred at room temperature for 2-3 hours, then is heated at 90°C until a homogenous solution results and formation of basic vapours is finished.

The solution, after cooling in ice bath is neutralised with diluted HCl 1:1. The precipitate is collected on a fritted glass filter and dried in a desiccator.

### Example 32: synthesis of sulfoindodicarbo-µ-(4-pyridyl)cyanine sodium salt (Compound 32)

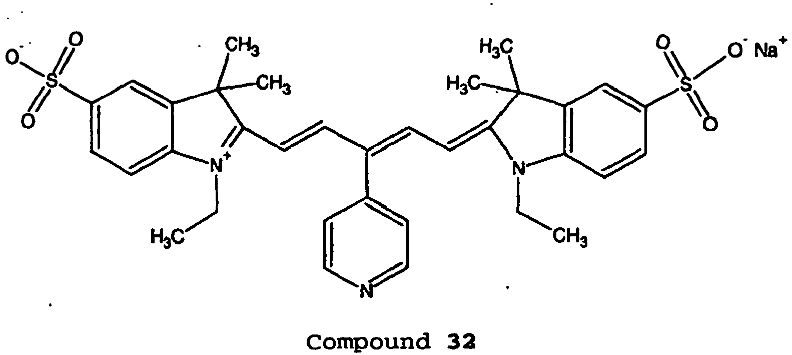

0.5 g of N-ethyl-2,3,3-trimethyl-3[H]indolium-5-sulfonate, 0.14 g of compound **31,** 1 ml of dry pyridine and 20 ml of acetic anhydride in a 100 ml flask are heated at reflux for a hour. The blue solution is cooled to room temperature and added dropwise to 200 ml of rapidly stirred diethyl ether. The blue solid is collected on fritted glass filter, washed with ether and dried in a desiccator. The product has λ_{MeOH}= 642 nm.

### Example 33: synthesis of sulfoindodicarbo-µ-[N-(5-carboxypenthyl)-4-pyridinium) cyanine (Compound 33)

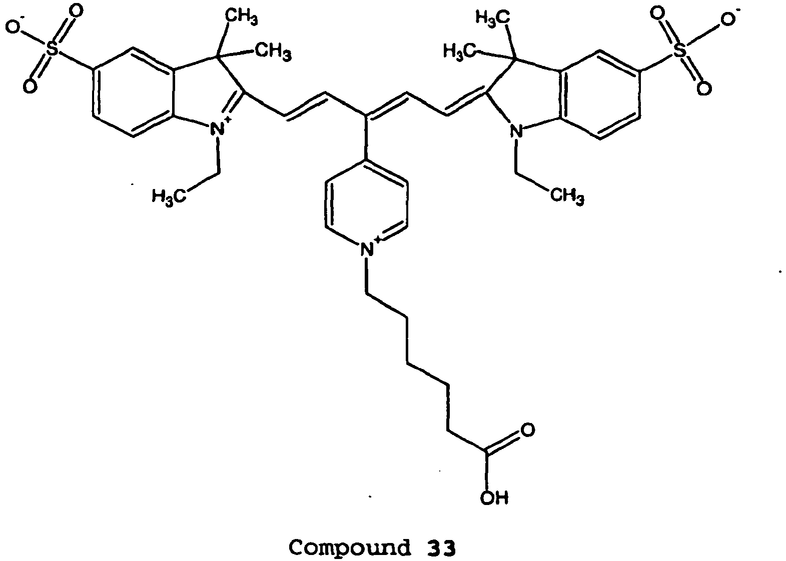

0.3 g of compound **32,** 0.27 g of 6-bromohexanoic acid, 0.118 g of N-ethyldiisopropylamine and 20 ml of dry DMF in a 100 ml flask are heated, under argon, at 120°C for 4 hours.

The blue solution is cooled to room temperature and added dropwise to 200 ml of rapidly stirred diethyl ether. The blue solid is collected on fritted glass filter, washed with ether and dried in a desiccator. The product has λMeOH=638 nm.

## Claims

1. A symmetric cyanine of the formula: 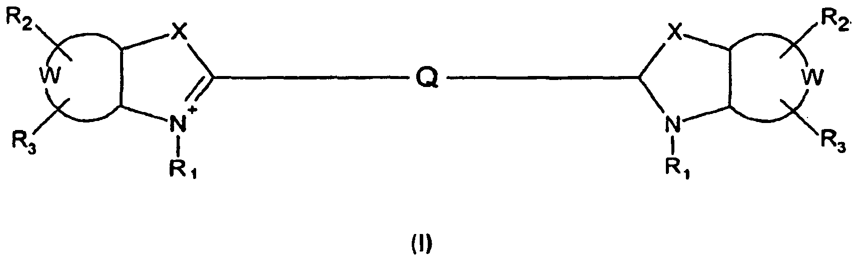 wherein:
X is selected from the group consisting of 0, S and C(CH₃)₂;
W represents non-metal atoms required to form a benzo-condensed or a naphtho-condensed ring;
R1 is selected from the group consisting of (CH₂)ₙCH₃, (CH₂)ₙSO₃⁻ and (CH₂)ₙSO₃H,
wherein n is an integer selected from 0 to 6 when R1 is (CH₂)ₙCH₃, and n is an integer selected from 3 to 6 when R1 is (CH₂)ₙSO₃⁻ or (CH₂)ₙSO₃H;
R₂ and R₃ are independently selected from the group consisting of H, a sulphonic moiety and a sulphonate moiety;
Q is selected from the group consisting of: 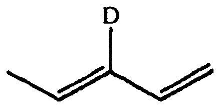 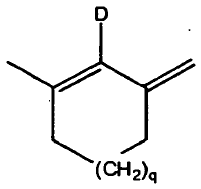 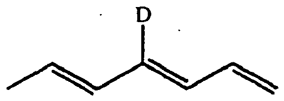 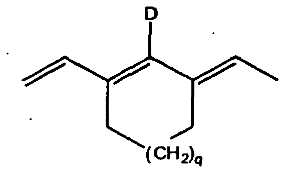 and 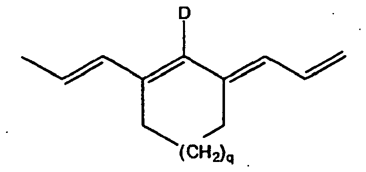 wherein q is 0 or 1 and D is selected from the group consisting of:
-C≡C-G; 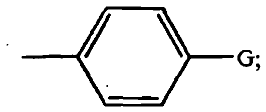
and 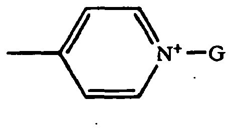 wherein
G is a nucleophile moiety selected from the group consisting of (CH₂)ₘNH₂, (CH₂)ₘSH, (CH₂)ₘY(CH₂)ₚOH, (CH₂)ₘY(CH₂)ₚNH₂ and (CH₂)ₘY(CH₂)ₚSH, wherein Y is selected from the group consisting of -NH-, -CONH-, -O- and -S-, m is an integer selected from 0 to 6 and p is an integer selected from 1 to 6;
or wherein G is a moiety capable of reacting with N, O or S nucleophiles, and is selected from the group consisting of (CH₂)ₘCOOH, (CH₂)ₘglycidyl, (CH₂)ₘmaleimide, (CH₂)ₘCO-NHS, (CH₂)ₘCO-imidazole, (CH₂)ₘSO₂CH=CH₂, (CH₂)ₘCONHNH₂, (CH₂)ₘCHO, (CH₂)ₘY(CH₂)ₚCOOH, (CH₂)ₘY(CH₂)ₚglycidyl, (CH₂)ₘY(CH₂)ₚmaleimide, (CH₂)ₘY(CH₂)ₚCO-NHS, (CH₂)ₘY(CH₂)ₚCO-imidazole, CH₂(CH₂)ₘO-PAM, (CH₂)ₘY(CH₂)ₚSO₂CH=CH₂, (CH₂)ₘY(CH₂)ₚCONHNH₂, (CH₂)ₘY(CH₂)ₚCHO and (CH₂)ₘY(CH₂)ₚO-PAM, wherein Y, m and p have the meanings indicated above, where
NHS is 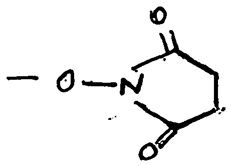 and PAM is

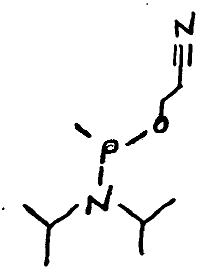

2. A symmetric cyanine according to claim 1, wherein at least one of the moieties R₁ to R₃ is, or contains a sulphonic moiety or a sulphonate moiety.

3. A symmetric cyanine according to claim 1 or 2, wherein X is C(CH₃)₂.

4. A symmetric cyanine according to claim 3, wherein one of the moieties R₂ and R₃ is a sulphonic moiety or a sulphonate moiety.

5. A symmetric cyanine according to claim 4, wherein R₁ is (CH₂)ₙSO₃⁻ or (CH₂)ₙSO₃H.

6. A symmetric cyanine according to claim 1 or 2, wherein X is S and R₁ is (CH₂)ₙSO₃ or (CH₂)ₙSO₃H.

7. A symmetric cyanine according to claim 1 to 6 having any of the formulae 2a to 21: 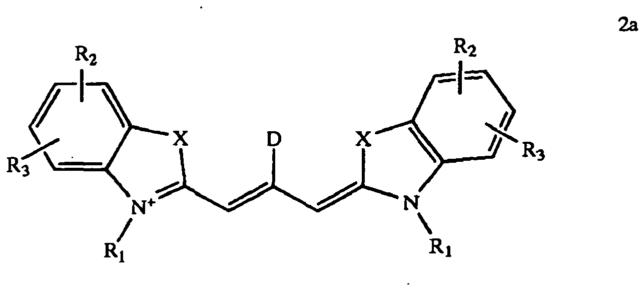 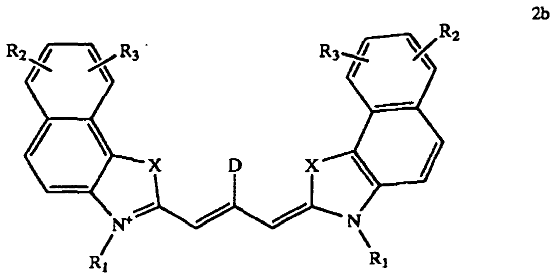 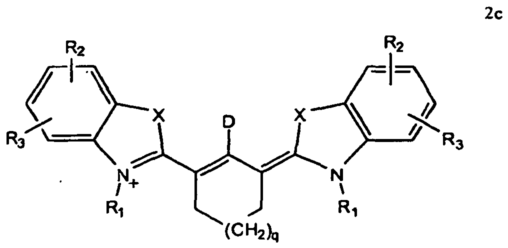 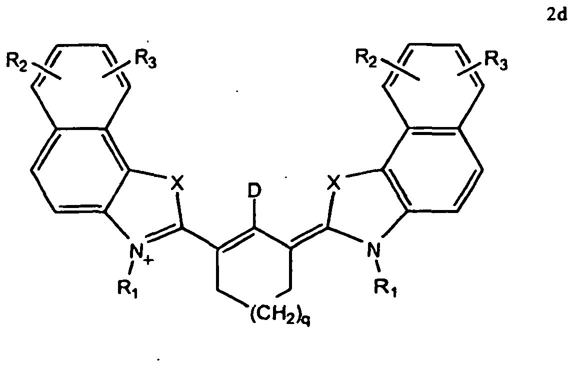 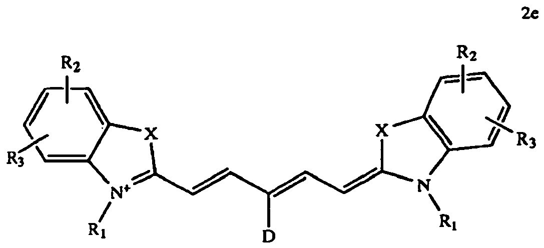 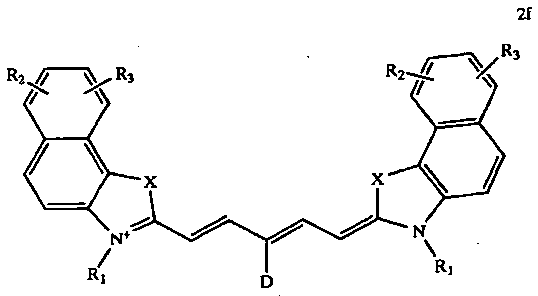 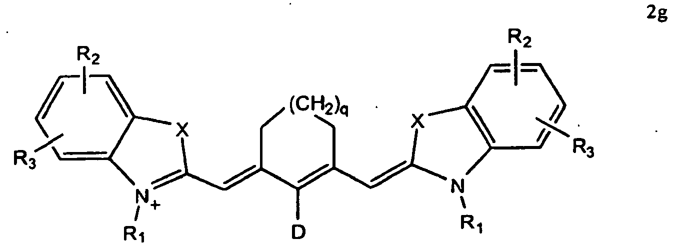 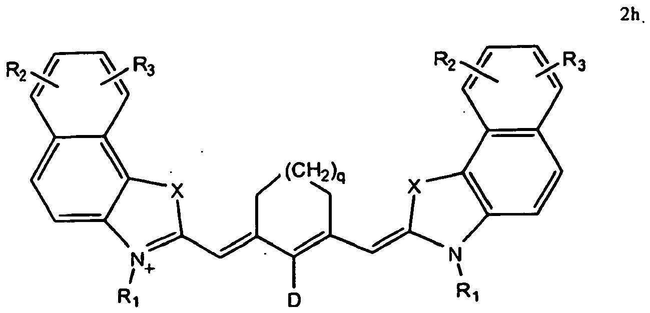 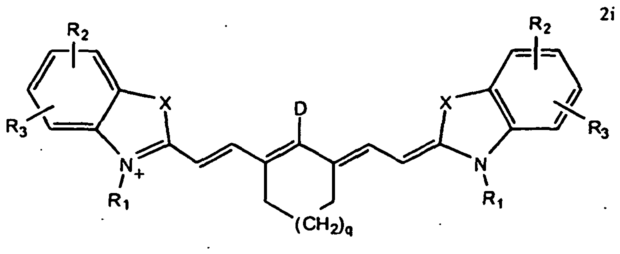 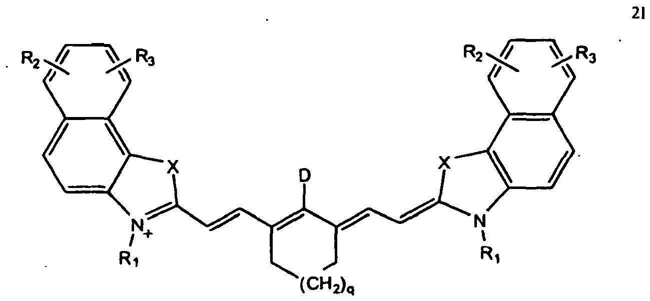 wherein R₁, R₂, R₃, X, q and D have the meanings indicated in claim 1.

## Patentansprüche

1. Ein symmetrisches Cyanin mit der Formel: 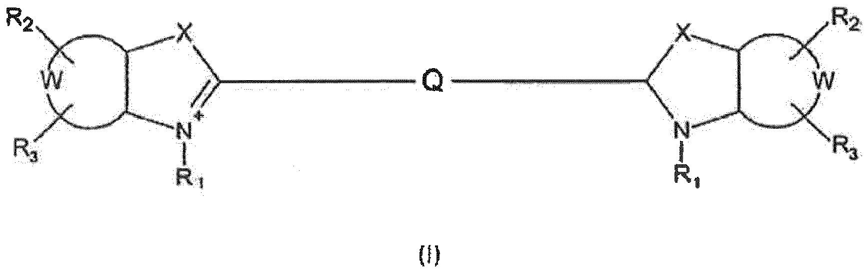 wobei
X aus der Gruppe gewählt wird, die aus O, S und C(CH₃)₂ besteht;
W nichtmetallischen Atomen entspricht, die zur Bildung eines benzokondensierten oder eines naphthokondensierten Rings erforderlich sind;
R₁ aus der Gruppe gewählt wird, die aus (CH₂)ₙCH₃, (CH₂)ₙSO₃ und (CH₂)ₙSO₃H besteht; hierbei ist n eine ganze Zahl von 0 bis 6, wenn es sich bei R₁ um (CH₂)ₙCH₃ handelt, und n eine ganze Zahl von 3 bis 6, wenn R₁ gleich (CH₂)ₙSO₃ oder (CH₂)ₙSO₃H ist.
R₂ und R₃ werden unabhängig voneinander aus der Gruppe gewählt, die aus H sowie einem Sulfon- und einem Sulfonatanteil besteht;
Q wird aus der Gruppe gewählt, die aus 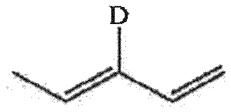 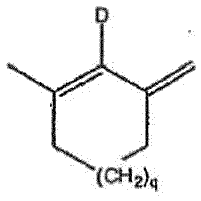 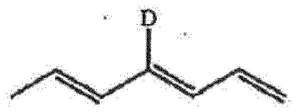 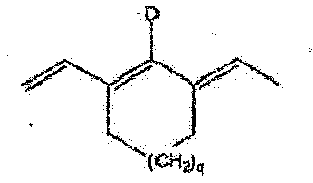 und 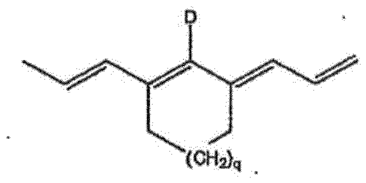 besteht, wobei q gleich 0 oder 1 ist, und D aus der Gruppe gewählt wird, die aus 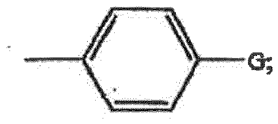 und 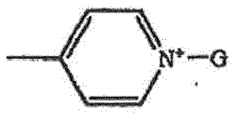 besteht, wobei
G eine nukleophile Einheit ist, die aus der Gruppe gewählt wird, die aus (CH₂)ₘNH₂, (CH₂)ₘSH, (CH₂)ₘY(CH₂)ₚOH, (CH₂)ₘY(CH₂)ₚNH₂ und (CH₂)ₘY(CH₂)ₚSH besteht, wobei Y aus der Gruppe gewählt wird, die aus -NH-, -CONH-, -O- und -S- besteht, m eine ganze Zahl von 0 bis 6 und p eine ganze Zahl von 1 bis 6 ist;
oder wobei G für eine Einheit steht, die mit N-, O- oder S-Nukleophilen reagieren kann und aus der Gruppe gewählt wird, die aus (CH₂)ₘCOOH, (CH₂).Glycidyl, (CH₂)ₘMaleimid, (CH₂)ₘCO-NHS, (CH₂)ₘCO-Imidazol, (CH₂)ₘSO₂CH = CH₂, (CH₂)ₘCONHNH₂, (CH₂)ₘCHO, (CH₂)ₘY(CH₂)_{P}COOH, (CH₂)ₘY(CH₂)_{P}Glycidyl, (CH₂)ₘY(CH₂)_{P}maleimid, (CH₂)ₘY(CH₂)_{P}CO-NHS, (CH₂₎ₘY(CH₂)_{P}CO-Imidazol, CH₂(CH₂)ₘO-PAM, (CH₂)ₘY(CH₂)_{P}SO₂CH = CH₂, (CH₂)ₘY(CH₂)_{P}CONHNH₂, (CH₂)ₘY(CH₂)_{P}CHO und (CH₂)ₘY(CH₂)_{P}O-PAM besteht, wobei Y, m und p oben angegebene Bedeutungen haben, wo
NHS ist 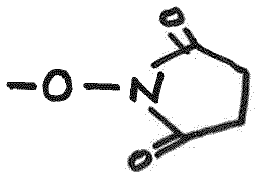 und PAM ist

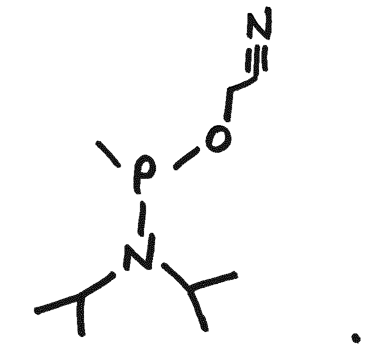

2. Ein symmetrisches Cyanin nach Anspruch 1, wobei mindestens eine der Einheiten R₁ bis R₃ ein Sulfon- bzw. Sulfonatanteil ist oder einen Sulfon- bzw. Sulfonatanteil enthält.

3. Ein symmetrisches Cyanin nach Anspruch 1 oder 2, wobei X gleich C(CH₃) ₂ ist.

4. Ein symmetrisches Cyanin nach Anspruch 3, wobei es sich bei einer der Einheiten R₂ und R₃ um einen Sulfon- bzw. einen Sulfonatanteil handelt.

5. Ein symmetrisches Cyanin nach Anspruch 4, wobei R₁ gleich (CH₂)ₙSO₃ oder (CH₂)ₙSO₃H ist.

6. Ein symmetrisches Cyanin nach Anspruch 1 oder 2, wobei X gleich S und R₁ gleich (CH₂)ₙSO₃ oder (CH₂)ₙSO₃H ist.

7. Ein symmetrisches Cyanin nach Anspruch 1 bis 6 mit einer der Formeln 2a bis 21: 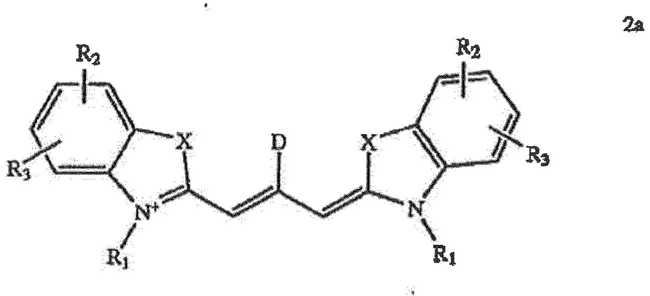 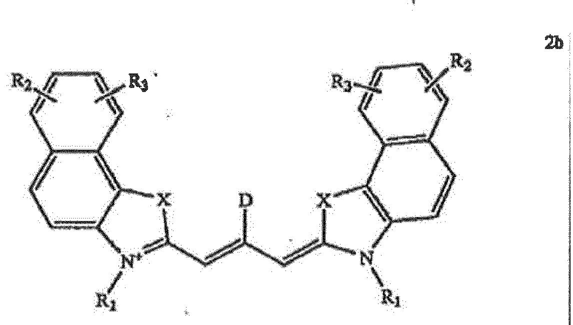 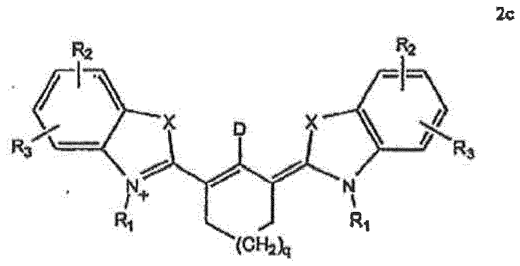 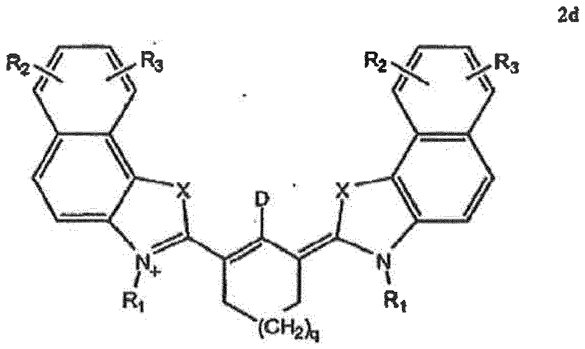 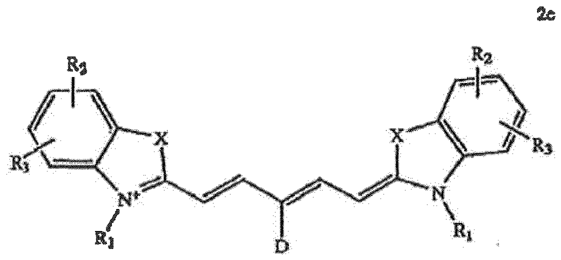 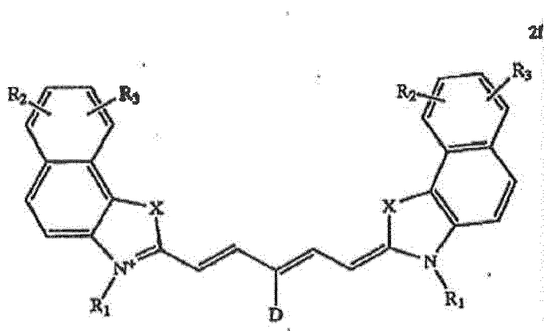 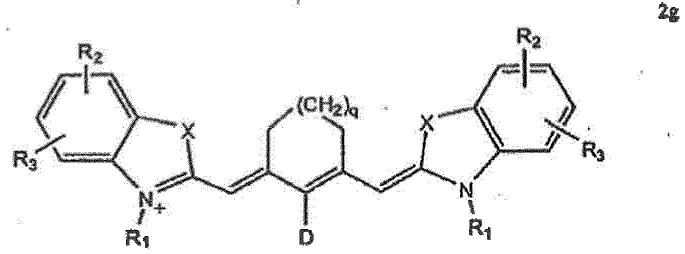 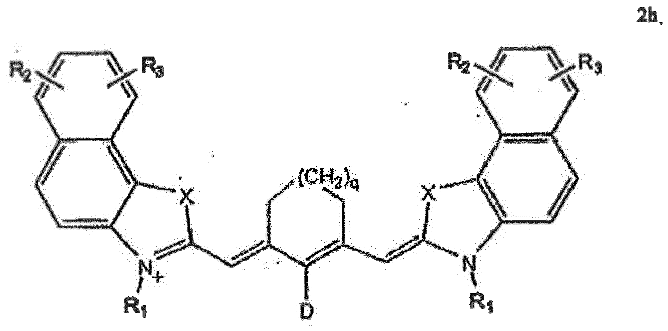 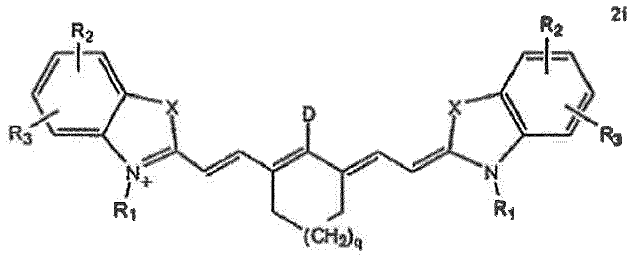 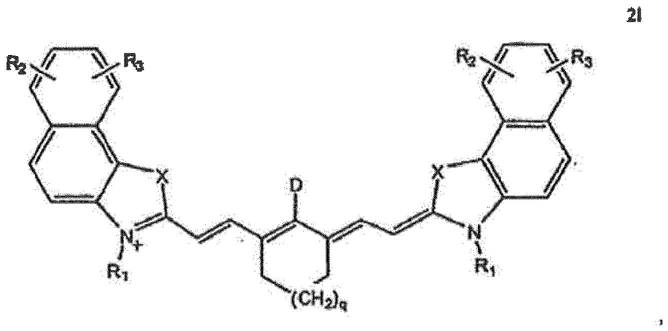 wobei R₁, R₂, R₃, X, q und D die in Anspruch 1 angegebenen Bedeutungen haben.

## Revendications

1. Cyanine symétrique de formule: 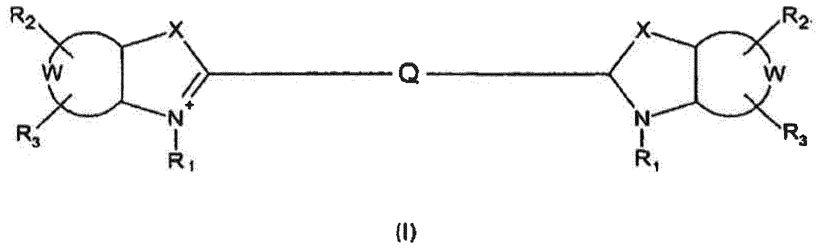 où:
X est choisi dans le groupe consistant en O, S et C(CH₃)₂;
W représente des atomes non métalliques nécessaires pour former un cycle benzo-condensé ou naphto-condensé;
R₁ est choisi dans le groupe consistant en (CH₂)ₙCH₃, (CH₂)ₙSO₃⁻ et
(CH₂)ₙSO₃H, où n est un entier choisi parmi 0 à 6 quand R₁ est (CH₂)ₙCH₃, et n est un entier choisi parmi 3 à 6 quand R₁ est (CH₂)ₙSO₃⁻ ou (CH₂)ₙSO₃H;
R₂ et R₃ sont choisis indépendamment dans le groupe consistant en H, un groupement sulfonique et un groupement sulfonate ;
Q est choisi dans le groupe consistant en: 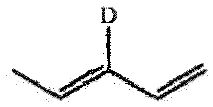 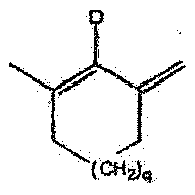 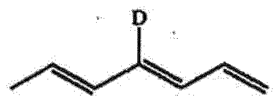 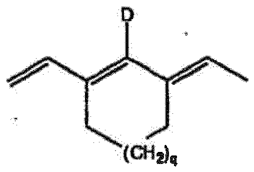 et 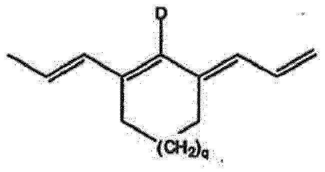 où q est 0 ou 1 et D est choisi dans le groupe consistant en:
-C≡C-G; 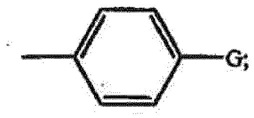
et 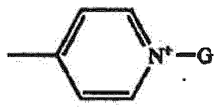 où
G est un groupement nucléophile choisi dans le groupe consistant en (CH₂)ₘNH₂, (CH₂)ₘSH, (CH₂)ₘY(CH₂)ₚOH, (CH₂)ₘY(CH₂)ₚNH₂ et (CH₂)ₘY(CH₂)ₚSH, où Y est choisi dans le groupe consistant en -NH-, -CONH-, -O- et -S-, m est un entier choisi parmi 0 à 6 et p est un entier choisi parmi 1 à 6;
ou bien où G est un groupement capable de réagir avec des nucléophiles N, O ou S, et est choisi dans le groupe consistant en (CH₂)ₘCOOH, (CH₂)ₘglycidyle, (CH₂)ₘmaléimide, (CH₂)ₘCO-NHS, (CH₂)ₘCO-imidazole, (CH₂)ₘSO₂CH =CH₂, (CH₂)ₘCONHNH₂, (CH₂)ₘCHO, (CH₂)ₘY(CH₂)ₚCOOH, (CH₂)ₘY(CH₂)ₚglycidyle, (CH₂)ₘY(CH₂)ₚmaléimide, (CH₂)ₘY(CH₂)ₚCO-NHS, (CH₂)ₘY(CH₂)ₚCO-imidazole, CH₂(CH₂)ₘO-PAM, (CH₂)ₘY(CH₂)ₚSO₂CH=CH₂, (CH₂)ₘY(CH₂)ₚCONHNH₂, (CH₂)ₘY(CH₂)ₚCHO et (CH₂)ₘY(CH₂)ₚO-PAM, où Y, m et p ont les significations indiquées ci-dessus, où
NHS est 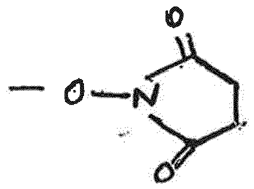 et PAM est

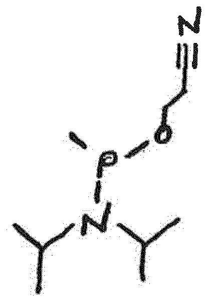

2. Cyanine symétrique selon la revendication 1, où au moins l'un des groupements R₁ à R₃ est, ou contient un groupement sulfonique ou un groupement sulfonate.

3. Cyanine symétrique selon la revendication 1 ou 2, où X est C(CH₃)₂.

4. Cyanine symétrique selon la revendication 3, où l'un des groupements R₂ et R₃ est un groupement sulfonique ou un groupement sulfonate.

5. Cyanine symétrique selon la revendication 4, où R₁ est (CH₂)ₙSO₃⁻ ou (CH₂)ₙSO₃H.

6. Cyanine symétrique selon la revendication 1 ou 2, où X est S et R₁ est (CH₂)ₙSO₃⁻ ou (CH₂)ₙSO₃H.

7. Cyanine symétrique selon les revendications 1 à 6 ayant l'une quelconque des formules 2a à 21 : 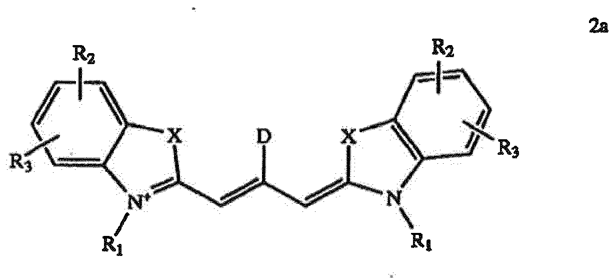 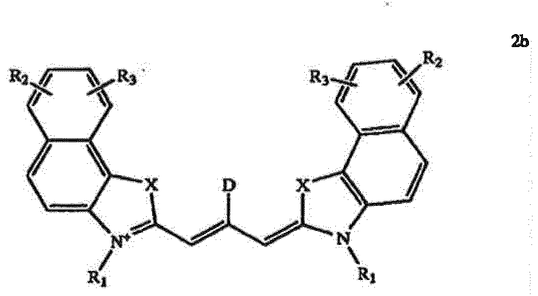 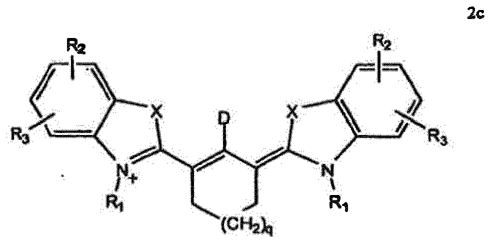 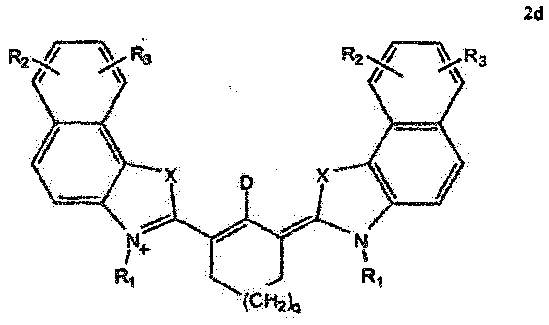 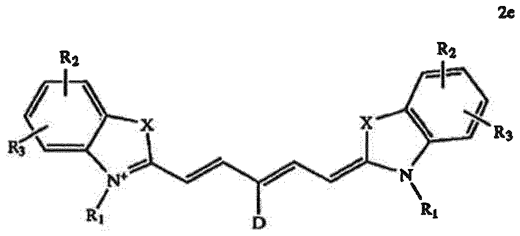 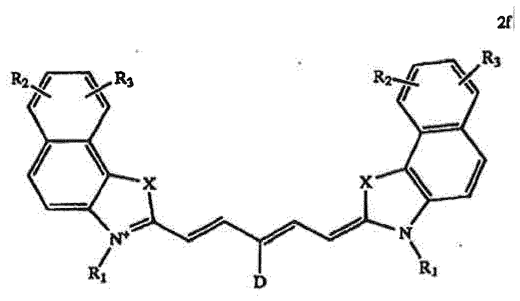 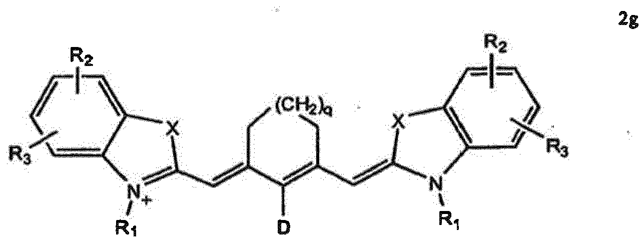 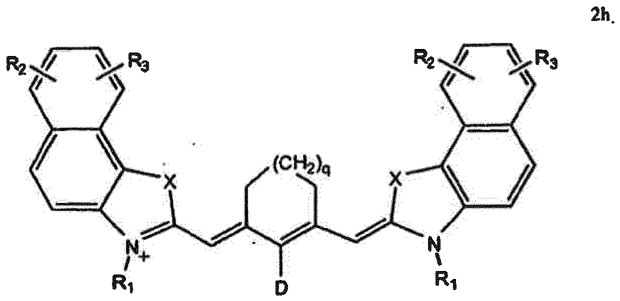 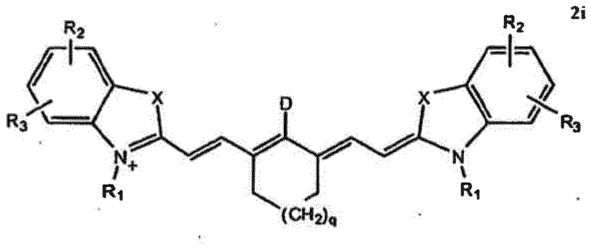 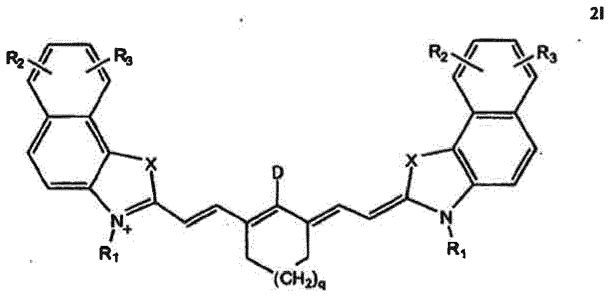 où R₁, R₂, R₃, X, q et D ont les significations indiquées dans la revendication 1.
